# EUROPEAN PATENT SPECIFICATION

(11) **EP 1 664 109 B1**
(45) Date of publication and mention of the grant of the patent: **22.07.2009**
(21) Application number: 04739040.6
(22) Date of filing: 18.08.2004
(51) Int. Cl.: C07K 14/605

(54) **PURIFICATION OF GLUCAGON-LIKE PEPTIDES**
REINIGUNG GLUCAGONÄHNLICHER PEPTIDE
PURIFICATION DE PEPTIDES APPARENTES AU GLUCAGON

(30) Priority: 21.08.2003 DK 200301197; 25.08.2003 US 497887 P
(43) Date of publication of application: 07.06.2006
(73) Proprietor: NOVO NORDISK A/S, 2880 Bagsvaerd (DK)
(72) Inventor: STABY, Arne, DK-2880 Bagsværd (DK); KORNBECK, Camilla, DK-3460 Birkerød (DK); DÜNWEBER, Dorte Lunoe, DK-2830 Virum (DK); CHRISTENSEN, Hanne, DK-2000 København F (DK); SCHOU, Ole, DK-4773 Stensved (DK)
(74) Representative: Noergaard, Torsten
(86) International application number: PCT/DK2004/000543
(87) International publication number: WO 2005/019262

(56) References cited:
- WO-A-00/55203
- US-A1- 2001 021 767
- US-A1- 2003 144 471
- THUM A ET AL.: "Endoproteolysis by isolated membrane peptidases reveal metabolic stability of glucagon-like peptide-1 analogs, exendins-3 and -4" EXPERIMENTAL AND CLINICAL ENDOCRINOLOGY AND DIABETES, vol. 110, no. 3, May 2002 (2002-05), pages 113-118, XP008040815

### SEQUENCE LISTING

<110> Novo Nordisk A/S
<120> Purification of glucagon-like peptides
<130> 6643.000-DK
<160> 7
<170> PatentIn version 3.1
<210> 1
   <211> 31
   <212> PRT
   <213> Homo sapiens
<400> 1
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Gly
               20                  25                  30
<210> 2
   <211> 33
   <212> PRT
   <213> Homo sapiens
<400> 2
   His Ala Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp
<210> 3
   <211> 39
   <212> PRT
   <213> Glia monster
<220>
   <221> MOD_RES
   <222> (39)..(39)
   <223> Serine at position 39 is amidated
<400> 3
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 4
   <211> 39
   <212> PRT
   <213> Gila monster
<220>
   <221> MOD_RES
   <222> (39)..(39)
   <223> Serine at position 39 is amidated
<400> 4
   His Ser Asp Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Pro Ser
   35
<210> 5
   <211> 37
   <212> PRT
   <213> Homo sapiens
<400> 5
   His Ser Gln Gly Thr Phe Thr Ser Asp Tyr Ser Lys Tyr Leu Asp Ser
   1 5 10 15
   Arg Arg Ala Gln Asp Phe Val Gln Trp Leu Met Asp Thr Lys Arg Asn
   20 25 30
   Lys Asn Asn Ile Ala
   35
<210> 6
   <211> 44
   <212> PRT
   <213> Artificial sequence
<220>
   <223> Synthetic construct
<220>
   <221> MOD_RES
   <222> (44)..(44)
   <223> Lysine at position 44 is amidated
<400> 6
   His Gly Glu Gly Thr Phe Thr Ser Asp Leu Ser Lys Gln Met Glu Glu
   1 5 10 15
   Glu Ala Val Arg Leu Phe Ile Glu Trp Leu Lys Asn Gly Gly Pro Ser
   20 25 30
   Ser Gly Ala Pro Pro Ser Lys Lys Lys Lys Lys Lys
   35 40
<210> 7
   <211> 33
   <212> PRT
   <213> Synthetic construct
<400> 7
   His Gly Asp Gly Ser Phe Ser Asp Glu Met Asn Thr Ile Leu Asp Asn
   1 5 10 15
   Leu Ala Ala Arg Asp Phe Ile Asn Trp Leu Ile Gln Thr Lys Ile Thr
   20 25 30
   Asp

## Description

### FIELD OF THE INVENTION

The present invention relates to the field of protein purification. In particular, the invention relates to a method for purifying a glucagon-like peptide from a composition comprising the glucagon-like peptide and at least one related impurity by reversed phase high performance liquid chromatography.

### BACKGROUND OF THE INVENTION

For the purification and analysis of proteins and peptides (polypeptides), chromatography is a well-known and widely used method. A number of different chromatographic principles are applied, among these reversed phase high performance liquid chromatography (RP-HPLC). The RP-HPLC separation principle is based on hydrophobic association between the polypeptide solute and hydrophobic ligates on the chromatographic resin surface. RP-HPLC purification usually consists of one or more of the following sections: equilibration, loading, wash, elution, and regeneration.

The most commonly applied solvent system in RP-HPLC is based on water/acetonitrile/tri-fluoro-acetic acid (TFA), and elution of solutes is usually accomplished by increasing organic content, i.e. acetonitrile, of the liquid applied to the chromatographic column. Acetonitrile has a strong selective and denaturating effect on polypeptide solutes in RP-HPLC (Boysen, R.I. et al., J. Biol. Chem. 277, 23-31 (2002)) and combined with TFA (consequently at low pH - 2), this system is applied as a standard analytical tool in the pharmaceutical industry and other industries (Snyder, L.R. et al., "Practical HPLC method development", 2nd ed., chapter 11 in "Biochemical samples: Proteins, nucleic acids, carbohydrates, and related compounds", John Wiley&Sons Inc., New York, 1997). Also in production scale has acetonitrile at low pH been used widely for polypeptide purification, i.e. for purification of human insulin (Kroeff, E.P. et al., J. Chromatogr. 461, 45-61 (1989)). An unsubstituted polymer-based reversed phase resin has been used for the initial recovery of glucagon from pancreas glands (US 4,617,376). The chromatographic column was operated at pH 2.8 with acetonitrile as the organic solvent and glycine as the buffer component. There were no indications of removal of related impurities by this step. Various glucagon analogues obtained from peptide synthesis were purified on a C₁₈-column with a linear gradent in an acetonitrile/TFA system at low pH (Krstenansky, J.L. et al., J. Biochem. 25, 3839-3845 (1986)). Glucagon has been isolated from elasmobranchian fish on a C₁₈-column using a linear acetonitrile gradient at low pH employing TFA as buffer substance (Conlon J.M. and Thim L. Gen. Comp. Endocrinol. 60, 398-405 (1985)). Recombinant chicken glucagon was expressed in *E*. *coli* and subsequently purified using various steps including RP-HPLC with a linear gradent in an acetonitrile/TFA system at low pH (Kamisoyama H. et al. Anim. Sci. J. 71, 428-431 (2000)).

Insulin and glucagon have been separated from elephant fish on a C₁₈-column using a linear acetonitrile gradient at pH 7.65 employing 50 mM ammonium acetate as buffer system (Berks B.C., et al., Biochem. J. 263, 261-266 (1989)).

WO 99/52934 discloses a RP-HPLC method for separation of various insulin derivatives, where improved separation between target components and glycosylated, related impurities was achieved by addition of calcium ions. Purification was performed at 22-25°C using ethanol as the organic solvent, and Tris or Bis-Tris as buffer component in the pH range of approx. 7.0-7.2, that is above the isoelectric point of insulins.

A purification process for insulin including RP-HPLC steps on C₁₈-columns with ethanol as organic elution agent at both low pH using ammonium sulphate buffer and at pH close to neutral using Tris buffer has also been described (Mollerup I. et al., "Insulin purification" in "Encyclopedia of bioprocess technology", Eds. Flickinger M.C. and Drew S.W., pp 1491-1498, John Wiley&Sons Inc. 1999). Insulin related impurities were removed by these methods.

On a C₁₈-column separation has been obtained of iodinated glucagon products using various gradients starting with 40% methanol in water with 10 mM phosphate and triethylamine buffer at pH 3.0, and ending at either 50% of (acetonitrile/0.1 M ammoniumcarbonate, pH 9.0) or ending at 12.5% of (acetonitrile/0.1 M Tris-HCl, pH 9.0) (Rojas F.J. et al., Endo. 113, 711-719 (1983)). The glucagon products were separated by this mixed mode RP-HPLC (of both solvents and pH) according to degree of iodination. In addition, the methods were used to separate enzymatic digests of glucagon and iodinated glucagon.

As is the case for many other polypeptides, glucagon-like peptides including analogues and derivatives have been widely purified using RP-HPLC applying a linear gradient of acetonitrile with small amounts of TFA as buffer substance at low pH, that is, below the isoelectric point (pl) of the target polypeptide component. GLP-1 has been isolated from small intestines from two species, pigs and humans (Ørskov C. et al., J. Biol. Chem. 264, 12826-12829 (1989)). Purification was obtained using a linear gradient in an ethanol/TFA system, and additional purification was obtained using an isocratic elution in an acetonitrile/TFA system, both at low pH on a C₁₈-column. The two related GLP-1 forms present (GLP-1 and NH₂-terminally extended GLP-1) were not separated by either method.

An acetonitrile/TFA based RP-HPLC system has been applied for investigation of dog GLP-1 forms in ileum (Namba M. et al., Biomedical Res. 11(4), 247-254 (1990)). There were some indications that various forms were separated, and that synthetically obtained GLP-1 and des-Gly³⁷-GLP-1 amide standards had slightly different elution times applying this method. A C₄-column in an acetonitrile/TFA based RP-HPLC system at low pH has been applied for purification fusion proteins of a GLP-1 derivative and of exendin-4 with antibody fragments and human serum albumin (WO 02/46227).

Various preproglucagon cleavage products have been separated on a C₁₈-column with gradent elution in an acetonitrile/TFA system at low pH (Noe B.D. and Andrews P.C., Peptides 7, 331-336 (1986)).

A cyanopropyl column in an acetonitrile/TFA based RP-HPLC system at low pH has been used for purification of various GLP-1 analogues obtained from chemical synthesis (WO 98/08871).

GLP-2 has been separated from other proglucagon related peptides from intestinals from two species, pigs and humans (Buhl T. et al., J. Biol. Chem. 263, 8621-8624 (1988)). Purification was obtained using a linear gradient in an acetonitrile/TFA system at low pH, and additional purification was applied using an isocratic elution in an ethanol/TFA system, both at low pH on a C₁₈-column. By the latter method, cytochrome C oxidase was separated from GLP-2, however, the two related GLP-2 forms present (GLP-2 and NH₂-terminally extended GLP-2) were not separated.

WO 01/04156 discloses exendin-4 variants and GLP-1 variants obtained both synthetically and by recombinant technology. Variants obtained from peptide synthesis were purified on a C₁₈-column applying gradient elution of an acetonitrile/TFA system at low pH, while recombinant peptides were purified on a C₈-column applying a linear gradient of an acetonitrile/TFA system at low pH.

WO 00/41548 discloses the use of a C₁₈-column applying gradient elution of an acetonitrile/TFA system at low pH to purify exendin-3 and exendin-4 obtained from peptide synthesis. WO 99/25727 discloses the use of a C₁₈-column applying gradient elution of an acetonitrile/TFA system at low pH to purify various exendin agonists (exendin analogues and derivatives) obtained from peptide synthesis.

Glucagon, GLP-1, and GLP-2 from human pancreas extracts have been separated on a C₁₈-column using a linear gradient in an acetonitrile/TFA system at low pH (Suda K. et al., Biomedical Res. 9, 39-45 (1988)).

Flow rate and temperature effects have been disclosed for a RP-HPLC purification of a GLP-1 analogue obtained from recombinant technology on a C₁₈-column with ethanol as organic elution agent without controlling pH of the chromatographic solvents (Schou O., presented at 6th Interlaken Conference on Advances in Production of Biologicals, Interlaken, Switzerland, March 25-28, 2003).

EP 0708179 discloses the use of solid phase synthesis to generate various GLP-1 analogues and derivatives. One purification protocol employed included purification on a C₁₈-column at 45°C using a linear gradient in an acetonitrile/TFA system at low pH. Another purification protocol included two RP-HPLC steps at ambient temperature: purification on a C₄-column using a linear gradient in an acetonitrile/TFA system at low pH followed by purification on a C₁₈-column using a linear gradient in an acetonitrile/ammonium carbonate system at pH 7.7. Various related impurities and starting materials were removed by the two step method resulting in a HPLC purity of approx. 99% of the target component and an overall yield of only 14.8%.

Senderoff et al. (J. Pharm. Sci. 87, 183-189 (1998)) used solid phase synthesis and recombinant technology using expression in yeast to generate native human GLP-1 for studies of conformational changes. The purification protocol for the recombinant GLP-1 included among others two RP-HPLC steps using ethanol as the organic elution agent. The first RP-HPLC step was performed at pH 10.7 with 0.05 M ammonium hydroxide as buffer, while the second RP-HPLC step was performed at low pH (below pH 3) with 1 % acetic acid as buffer. The purification protocol resulted in a GLP-1 purity of approx. 98.5%, however, the product suffered from dramatic conformational changes resulting in difficulties in redissolution of the product. In addition, the high pH involved in process steps including the first RP-HPLC step induced base-catalyzed degradation products, that were less bioactive than the target compound. A third RP-HPLC step was employed (conditions not specified) as one of several steps to reprocess the target GLP-1 and bring it back to the right conformational structure.

US 2003/144471 regards a method for producing recombinant GLP-1 analogues and derivatives, using a reversed phase HPLC method for purification in a linear gradient buffer system comprising Tris, sodium sulphate and ethanol.

The present invention is based on the application of pH-buffered solvents comprising an alcohol as the organic elution agent for RP-HPLC purification of glucagon-like peptides and analogues and derivatives thereof at pH close to neutral. The present invention facilitates increased separation efficiency and application for industrial use compared to the current state of the art within RP-HPLC purification of glucagon-like peptides using alcohol-based solvent systems. Surprisingly, separation of target glucagon-like peptide compounds and related impurities is improved by the new methodology and results in more stable glucagon-like peptide products.

The use of pH close to neutral during RP-HPLC purification has the advantage that potential aggregation is avoided on the column for these glucagon-like peptides, which will be reflected by an example. This is surprising, because insulin and glucagon as presented above may be handled at low pH without aggregation on the column, hereby presenting the difference in nature between insulin and glucagon on one side and glucagon-like peptides on the other side.

The use of alcohol during RP-HPLC purification has the additional advantage of inducing better conformational conservation of peptides compared to the more commonly used acetonitrile. Further, acetonitrile (and TFA) are toxic chemicals, which due to environmental and health issues, are not suitable and should be avoided for use in industrial scale. Alcohols are generally less toxic and more suitable for industrial use.

### BREIF DESCRIPTION OF THE DRAWINGS.

Figure 1. Chromatogram of AU₂₈₀ versus time for the preparative separation using C₄-substituted 120Å silica gel and elution at pH 3.5 of Arg³⁴-GLP-1 (7-37) from related impurities which are glycosylated impurities.
Figure 2. Chromatogram of AU₂₈₀ versus time for the preparative separation using C₄-substituted 120Å silica gel and elution at pH 7.5 of Arg³⁴-GLP-1 (7-37) from related impurities which are glycosylated impurities as well as the truncated form, Arg³⁴-GLP-1 (9-37).
Figure 3. Chromatogram of AU₂₈₀ versus time for the preparative separation using C₁₈-substituted 200Å silica gel and elution at pH 3.5 of Arg³⁴-GLP-1 (7-37) from related impurities which are glycosylated impurities.
Figure 4. Chromatogram of AU₂₈₀ versus time for the preparative separation using C₁₈-substituted 200Å silica gel and elution at pH 7.5 of Arg³⁴-GLP-1 (7-37) from related impurities which are glycosylated impurities as well as the truncated form, Arg³⁴-GLP-1 (9-37).
Figure 5. Chromatogram of AU₂₈₀ versus time for the preparative separation using C₁₈-substituted 120Å silica gel and elution at pH 7.5 of Arg³⁴-GLP-1 (7-37) from related impurities which are glycosylated impurities as well as the truncated form, Arg³⁴-GLP-1(9-37).
Figure 6. Chromatogram of AU₂₈₀ versus time for the preparative separation of Arg³⁴-GLP-1(7-37) from related impurities which are glycosylated impurities using C₄-substituted 120Å silica gel and elution at pH 7.5 in a solvent without pH-buffer.

### DEFINITIONS

The following is a detailed definition of the terms used in the specification.

The term "purifying" a peptide from a composition comprising the peptide and one or more contaminants means increasing the degree of purity of the peptide in the composition by reducing the contents of at least one contaminant from the composition.

The term "related impurity" as used herein means an impurity which has structural resemblance to the target glucagon-like peptide. A related impurity has different chemical or physical structure than the target glucagon-like peptide, and is a truncated form, an extended form (extra amino acids, various derivatives etc.), a deamidated form, an incorrectly folded form, a form with undesired glycosylation including sialylation, oxidated forms, forms resulting from racemization, forms lacking amino acids in the intra-peptide chain, forms having extra amino acids in the intra-peptide chain, forms wherein an acylation has taken place on another residue than desired.

The term "buffer" as used herein refers to a chemical compound that reduces the tendency of pH of a chromatographic solvent to change over time as would otherwise occur. Buffers include but are not limited to chemicals such as sodium acetate, sodium carbonate, sodium citrate, glycylglycine, glycine, histidine, lysine, sodium phosphate, borate, TRIS (Tris-hydroxymethyl-aminomethane), ethanolamine or mixtures thereof.

The term "glucagon-like peptide" as used herein refers to the homologous peptides glucagon-like peptide 1 (GLP-1), glucagon-like peptide 2 (GLP-2), and oxynthomodulin (OXM) derived from the preproglucagon gene, the exendins as well as analogues and derivatives thereof. The exendins which are found in the Gila monster are homologous to GLP-1 and also exert an insulinotropic effect. Examples of exendins are exendin-4 and exendin-3.

The glucagon-like peptides have the following sequences (SEQ ID Nos 1-5) :

The term "analogue" as used herein referring to a peptide means a modified peptide wherein one or more amino acid residues of the peptide have been substituted by other amino acid residues and/or wherein one or more amino acid residues have been deleted from the peptide and/or wherein one or more amino acid residues have been deleted from the peptide and or wherein one or more amino acid residues have been added to the peptide. Such addition or deletion of amino acid residues can take place at the N-terminal of the peptide and/or at the C-terminal of the peptide. Two different and simple systems are often used to describe analogues : For example Arg³⁴-GLP-1(7-37) or K34R-GLP-1 (7-37) designates a GLP-1 analogue wherein the naturally occuring lysine at position 34 has been substituted with arginine (standard single letter abbreviation for amino acids used according to IUPAC-IUB nomenclature).

The term "derivative" as used herein in relation to a parent peptide means a chemically modified parent protein or an analogue thereof, wherein at least one substituent is not present in the parent protein or an analogue thereof, i.e. a parent protein which has been covalently modified. Typical modifications are amides, carbohydrates, alkyl groups, acyl groups, esters, pegylations and the like. An examples of a derivative of GLP-1 (7-37) is Arg³⁴, Lys²⁶(N^{ε}-(γ-Glu(N^{α}-hexadecanoyl)))-GLP-1 (7-37).

The term "a fragment thereof" as used herein in relation to a peptide means any fragment of the peptide having at least 20% of the amino acids of the parent peptide. Thus, for human serum albumin a fragment would comprise at least 117 amino acids as human serum albumin has 585 amino acids. In one embodiment the fragment has at least 35% of the amino acids of the parent peptide. In another embodiment the fragment has at least 50% of the amino acids of the parent peptide. In another embodiment the fragment has at least 75% of the amino acids of the parent peptide.

The term "variant" as used herein in relation to a peptide means a modified peptide which is an analog of the parent peptide, a derivative of the parent peptide or a derivative of an analog of the parent peptide.

The term "GLP-1 peptide" as used herein means GLP-1 (7-37), a GLP-1 analogue, a GLP-1 derivative or a derivative of a GLP-1 analogue.

The term "GLP-2 peptide" as used herein means GLP-2(1-33), a GLP-2 analogue, a GLP-2 derivative or a derivative of a GLP-2 analogue.

The term "exendin-4 peptide" as used herein means exendin-4(1-39), an exendin-4 analogue, an exendin-4 derivative or a derivative of an exendin-4 analogue.

The term "plasma stable glucagon-like peptide" as used herein means a chemically modified glucagon-like peptide, i.e. an analogue or a derivative which exhibits an in vivo plasma elimination half-life of at least 10 hours in man, as determined by the following method. The method for determination of plasma elimination half-life of a glucagon-like peptide in man is : The compound is dissolved in an isotonic buffer, pH 7.4, PBS or any other suitable buffer. The dose is injected peripherally, preferably in the abdominal or upper thigh. Blood samples for determination of active compound are taken at frequent intervals, and for a sufficient duration to cover the terminal elimination part (e.g. Pre-dose, 1, 2, 3, 4, 5, 6, 7, 8, 10, 12, 24 (day 2), 36 (day 2), 48 (day 3), 60 (day 3), 72 (day 4) and 84 (day 4) hours post dose). Determination of the concentration of active compound is performed as described in Wilken et al., Diabetologia 43(51):A143, 2000. Derived pharmacokinetic parameteres are calculated from the concentration-time data for each individual subject by use of non-compartmental methods, using the commercially available software WinNonlin Version 2.1 (Pharsight, Cary, NC, USA). The terminal elimination rate constant is estimated by log-linear regression on the terminal log-linear part of the concentration-time curve, and used for calculating the elimination half-life.

The term "DPP-IV protected glucagon-like peptide" as used herein means a glucagon-like peptide which has been chemically modified to render said peptide resistant to the plasma peptidase dipeptidyl aminopeptidase-4 (DPP-IV) than the native form of said peptide.

The term "immunomodulated exendin-4 peptide" as used herein means an exendin-4 peptide which is an analogue or a derivative of exendin-4(1-39) having a reduced immune response in humans as compared to exendin-4(1-39). The method for assessing the immune response is to measure the concentration of antibodies reactive to the exendin-4 peptide after 4 weeks of treatment of the patient.

The term "glucagon-like peptide product" as used herein means the purified peptide product which is to be used for the manufacture of a pharmaceutical composition. Thus, the glucagon-like peptide product is normally obtained as the product from the final purification, drying or conditioning step. The product may be crystals, precipitate, solution or suspension. The glucagon-like peptide product is also known in the art as the drug substance, i.e. the active pharmaceutical ingredient. The term "isoelectric point" as used herein means the pH value where the overall net charge of a macromolecule such as a polypeptide is zero. In polypeptides there may be many charged groups, and at the isoelectric point the sum of all these charges is zero. At a pH above the isoelectric point the overall net charge of the polypeptide will be negative, whereas at pH values below the isoelectric point the overall net charge of the polypeptide will be positive.

The term "pharmaceutical" as used herein with reference to a composition means that it is useful for treating a disease or disorder.

The term "pharmaceutically acceptable" as used herein means suited for normal pharmaceutical applications, i.e. giving rise to no adverse events in patients etc.

The term "effective amount" as used herein means a dosage which is sufficient to be effective for the treatment of the patient compared with no treatment.

The term "pharmaceutical composition" as used herein means a product comprising an active compound or a salt thereof together with pharmaceutical excipients such as buffer, preservative, and optionally a tonicity modifier and/or a stabilizer. Thus a pharmaceutical composition is also known in the art as a pharmaceutical formulation.

The term "excipients" as used herein means the chemical compounds which are normally added to pharmaceutical compositions, e.g. buffers, tonicity agents, preservatives and the like.

The term "treatment of a disease" as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder.

### DESCRIPTION OF THE INVENTION

In a first aspect the invention relates to a method for purifying a glucagon-like peptide from a composition comprising said glucagon-like peptide and at least one related impurity, which method is a reversed phase high performance liquid chromatographic process wherein the solvent used for elution is pH-buffered in the range from pH 4 to pH 10, said solvent is pH-buffered so as to prevent pH excursions of more than +/-1.0 pH units from the setpoint during the elution step, and said solvent comprises an alcohol in a concentration from 20% w/w to 60% w/w, wherein a related impurity is an impurity which has structural resemblance to the target glucagon-like peptide and is selected from the group consisting of a truncated form, an extended form, a deamidated form, an incorrectly folded form, a form with undesired glycosylation, an oxidated form, a form resulting from racemization, a form lacking amino acids in the intra-peptide chain, a form having extra amino acids in the intra-peptide chain and a form wherein an acylation has taken place on another residue than desired.

The target GLP moiety and impurities are eluted and separated by a step, asymptotic or linear change gradient or isocratically in organic solvent, or in combinations of these. The organic solvent component gradient would be from a lower to a higher concentration. Elution may also be possible by changing pH and/or temperature in the elution section.

The equilibration solution and the sample for application may or may not contain the organic solvent. The organic solvent could be but is not limited to any monohydric aliphatic alcohol (methanol, ethanol, propanols and butanols). Optional salt components for any section of the chromatographic purification may be any salt including but not limited to: NaCl, KCI, NH₄Cl, CaCl₂, sodium acetate, potassium acetate, ammonium acetate etc. Any buffer component can be used including but not limited to : Citrate buffers, phosphate buffers, TRIS buffers, borate buffers, carbonate buffers, acetate buffers, ammonium buffers, glycine buffers etc. The method also applies for any choice of chromatographic reversed phase resin optionally with any kind of substitution, including but not limited to : Silica based resin, such as Kromasil 100 C₁₈, polymer based resin such as Source from Amersham Biosciences, Poros materials from Applied Biosystems, e.g. Poros R1, R2 and R3 reversed phase resins, ceramic based resins from Ciphergen, metal oxide based resins, and others. Preferably, a silica based resin is used.

In one embodiment of the invention the solvent is pH-buffered in the range from about pH 5 to about pH 9.

In another embodiment of the invention the solvent is pH-buffered at a pH which is higher than the isoelectric point of said glucagon-like peptide.

In another embodiment of the invention the solvent is pH-buffered so as to prevent pH excursions of more than +/- 1.0 pH units from the setpoint during the elution step.

In another embodiment of the invention the solvent is pH-buffered so as to prevent pH excursions of more than +/- 0.5 pH units from the setpoint during the elution step.

In another embodiment of the invention the alcohol is ethanol.

In another embodiment of the invention the alcohol is 2-propanol.

In another embodiment of the invention the alcohol is selected from the group consisting of methanol, 1-propanol and hexylene glycol.

In another embodiment of the invention the reversed phase high performance liquid chromatographic process is performed using a silica based chromatographic resin.

In another embodiment of the invention the resin is a substituted silica gel, such as C₄-, C₆-, C₈-, C₁₂-, C₁₆-, C₁₈-, C₂₀-, phenyl- or benzene-substituted silica gel.

In another embodiment of the invention the reversed phase high performance liquid chromatographic process is performed using a chromatographic resin which is a polymeric base material.

The RP-HPLC methods with the increased selectivity of glucagon-like peptides close to neutral pH preferably apply to removal of related impurities with a different chemical or physical structure than the target glucagon-like peptide, and is selected from the group consisting of truncated forms, all kinds of extended forms (extra amino acids, various derivatives etc.), deamidated forms, incorrectly folded forms, forms with undesired glycosylation including sialylation, oxidated forms, forms resulting from racemization, forms lacking amino acids in the intra-peptide chain, forms having extra amino acids in the intra-peptide chain and others.

In one embodiment of the invention the related impurity is a truncated form of said glucagon-like peptide.

In another embodiment of the invention the related impurity is a glycosylated form of said glucagon-like peptide.

In another embodiment of the invention the solvent comprises an alcohol in a concentration from about 20%w/w to about 40%w/w.

In another embodiment of the present invention the glucagon-like peptide is GLP-1, a GLP-1 analogue, a derivative of GLP-1 or a derivative of a GLP-1 analogue.

In another embodiment of the present invention the GLP-1 analogue is selected from the group consisting of Arg³⁴-GLP-1(7-37), Gly⁸-GLP-1(7-36)-amide, Gly⁸-GLP-1(7-37), Val⁸-GLP-1 (7-36)-amide, Val⁸-GLP-1(7-37), Val⁸Asp²²-GLP-1(7-36)-amide, Val⁸Asp²²-GLP-1(7-37), Val⁸Glu²²-GLP-1(7-36)-amide , Val⁸Glu²²-GLP-1(7-37), Val⁸Lys²²-GLP-1(7-36)-amide, Val⁸Lys²²-GLP-1 (7-37), Val⁸Arg²²-GLP-1 (7-36)-amide, Val⁸Arg²²-GLP-1(7-37), Val⁸His²²-GLP-1(7-36)-amide, Val⁸His²²-GLP-1 (7-37), Val⁸Trp¹⁹Glu²²-GLP-1 (7-37), Val⁸Glu²²Val²⁵-GLP-1(7-37), Val⁸Tyr¹⁶Glu²²-GLP-1(7-37), Val⁸Trp¹⁶Glu²²-GLP-1(7-37), Val⁸Leu¹⁶Glu²²-GLP-1(7-37), Val⁸Tyr¹⁸Glu²²-GLP-1(7-37), Val⁸Glu²²His³⁷-GLP-1(7-37), Val⁸Glu²²Ile³³-GLP-1 (7-37), Val⁸Trp¹⁶Glu²²Val²⁵Ile³³-GLP-1(7-37), Val⁸Trp¹⁶Glu²²Ile³³-GLP-1(7-37), Val⁸Glu²²Val²⁵Ile³³-GLP-1 (7-37), Val⁸Trp¹⁶Glu²²Val²⁵-GLP-1(7-37), analogues thereof and derivatives of any of these.

In another embodiment of the present invention the derivative of GLP-1 or a derivative of a GLP-1 analogue has a lysine residue, such as one lysine, wherein a lipophilic substituent optionally via a spacer is attached to the epsilon amino group of said lysine.

In another embodiment of the present invention the lipophilic substituent has from 8 to 40 carbon atoms, preferably from 8 to 24 carbon atoms, e.g. 12 to 18 carbon atoms.

In another embodiment of the present invention the spacer is present and is selected from an amino acid, e.g. beta-Ala, L-Glu, or aminobutyroyl.

In another embodiment of the present invention the glucagon-like peptide is a DPPIV-protected glucagon-like peptide. The peptidase DPPIV hydrolyses glucagon-like peptides and the clearance rate of glucagon-like peptides may be reduced by those analogues of the natural forms of glucagon-like peptides which are DPPIV-protected, i.e. those analogues which under physiological conditions have a lower rate of hydrolysis by the DPPIV enzyme. In another embodiment of the present invention the glucagon-like peptide is a plasma stable glucagon-like peptide.

In another embodiment of the present invention the glucagon-like peptide is a derivative of a GLP-1 analogue which is Arg³⁴, Lys²⁶(N^{ε}-(γ-Glu(N^{α}-hexadecanoyl)))-GLP-1(7-37).

In another embodiment of the present invention the glucagon-like peptide is a GLP-1 peptide which has from 25 to 37 amino acid residues, preferable from 27 to 35 amino acid residues, even more preferable from 29 to 33 amino acid residues.

In one embodiment of the present invention the glucagon-like peptide is GLP-2, a GLP-2 analogue, a derivative of GLP-2 or a derivative of a GLP-2 analogue.

In another embodiment of the present invention the derivative of GLP-2 or a derivative of a GLP-2 analogue has a lysine residue, such as one lysine, wherein a lipophilic substituent optionally via a spacer is attached to the epsilon amino group of said lysine.

In another embodiment of the present invention the lipophilic substituent has from 8 to 40 carbon atoms, preferably from 8 to 24 carbon atoms, e.g. 12 to 18 carbon atoms.

In another embodiment of the present invention the spacer is present and is selected from an amino acid, e.g. beta-Ala, L-Glu, aminobutyroyl.

In another embodiment of the present invention the glucagon-like peptide has from 27 to 39 amino acid residues, preferable from 29 to 37 amino acid residues, even more preferable from 31 to 35 amino acid residues.

In another embodiment of the present invention the glucagon-like peptide is Gly²-GLP-2(1-33).

In one embodiment of the present invention the glucagon-like peptide is exendin-4, an exendin-4 analogue, a derivative of exendin-4, or a derivative of an exendin-4 analogue.

In another embodiment of the present invention the glucagon-like peptide is exendin-4.

In another embodiment of the present invention the glucagon-like peptide is the exendin-4 analogue ZP-10 (HGEGTFTSDLSKQMEEEAVRLFIEWLKNGGPSSGAPPSKKKKKK-NH2).

In another embodiment of the present invention the derivative of exendin-4 or derivative of an exendin-4 analogue is acylated or pegylated.

In another embodiment of the present invention the glucagon-like peptide is a stable exendin-4 peptide.

In another embodiment of the present invention the glucagon-like peptide is a DPP-IV protected exendin-4 peptide.

In another embodiment of the present invention the glucagon-like peptide is an immunomodulated exendin-4 peptide.

In another embodiment of the present invention the derivative of exendin-4 or derivative of an exendin-4 analogue has a lysine residue, such as one lysine, wherein a lipophilic substituent optionally via a spacer is attached to the epsilon amino group of said lysine.

In another embodiment of the present invention the lipophilic substituent has from 8 to 40 carbon atoms, preferably from 8 to 24 carbon atoms, e.g. 12 to 18 carbon atoms.

In another embodiment of the present invention the spacer is present and is selected from an amino acid, e.g. beta-Ala, L-Glu, or aminobutyroyl.

In another embodiment of the present invention the glucagon-like peptide is an exendin-4 peptide which has from 30 to 48 amino acid residues, from 33 to 45 amino acid residues, preferable from 35 to 43 amino acid residues, even more preferable from 37 to 41 amino acid residues.

In one embodiment of the invention the GLP-2 peptide is selected from the list consisting of: K30R-GLP-2(1-33); S5K-GLP-2(1-33); S7K-GLP-2(1-33); D8K-GLP-2(1-33); E9K-GLP-2(1-33); M10K-GLP-2(1-33); N11K-GLP-2(1-33); T12K-GLP-2(1-33); I13K-GLP-2(1-33); L14K-GLP-2(1-33); D15K-GLP-2(1-33); N16K-GLP-2(1-33); L17K-GLP-2(1-33); A18K-GLP-2(1-33); D21K-GLP-2(1-33); N24K-GLP-2(1-33); Q28K-GLP-2(1-33); S5K/K30R-GLP-2(1-33); S7K/K30R-GLP-2(1-33); D8K/K30R-GLP-2(1-33); E9K/K30R-GLP-2(1-33); M10K/K30R-GLP-2(1-33); N11K/K30R-GLP-2(1-33); T12K/K30R-GLP-2(1-33); I13K/K30R-GLP-2(1-33); L14K/K30R-GLP-2(1-33); D15K/K30R-GLP-2(1-33); N16K/K30R-GLP-2(1-33); L17K/K30R-GLP-2(1-33); A18K/K30R-GLP-2(1-33); D21 K/K30R-GLP-2(1-33); N24K/K30R-GLP-2(1-33); Q28K/K30R-GLP-2(1-33); K30R/D33K-GLP-2(1-33); D3E/K30R/D33E-GLP-2(1-33); D3E/S5K/K30R/D33E-GLP-2(1-33); D3E/S7K/K30R/D33E-GLP-2(1-33); D3E/D8K/K30R/D33E-GLP-2(1-33); D3E/E9K/K30R/D33E-GLP-2(1-33); D3E/M10K/K30R/D33E-GLP-2(1-33); D3E/N11 K/K30R/D33E-GLP-2(1-33); D3E/T12K/K30R/D33E-GLP-2(1-33); D3E/I13K/K30R/D33E-GLP-2(1-33); D3E/L14K/K30R/D33E-GLP-2(1-33); D3E/D15K/K30R/D33E-GLP-2(1-33); D3E/N16K/K30R/D33E-GLP-2(1-33); D3E/L17K/K30R/D33E-GLP-2(1-33); D3E/A18K/K30R/D33E-GLP-2(1-33); D3E/D21K/K30R/D33E-GLP-2(1-33); D3E/N24K/K30R/D33E-GLP-2(1-33); D3E/Q28K/K30R/D33E-GLP-2(1-33); and derivatives thereof.

In one embodiment of the invention the GLP-2 derivative is selected from the group consisting of
S5K(3-(hexadecanoylamino)propionyl)-GLP-2(1-33);
S7K(3-(hexadecanoylamino)propionyl)-GLP-2(1-33);
D8K(3-(hexadecanoylamino)propionyl)-GLP-2(1-33);
E9K(3-(hexadecanoylamino)propionyl)-GLP-2(1-33);
M10K(3-(hexadecanoylamino)propionyl)-GLP-2(1-33);
N11K(3-(hexadecanoylamino)propionyl)-GLP-2(1-33);
T12K(3-(hexadecanoylamino)propionyl)-GLP-2(1-33);
I13K(3-(hexadecanoylamino)propionyl)-GLP-2(1-33);
L14K(3-(hexadecanoylamino)propionyl)-GLP-2(1-33);
D15K(3-(hexadecanoylamino)propionyl)-GLP-2(1-33);
N16K(3-(hexadecanoylamino)propionyl)-GLP-2(1-33);
L17K(3-(octanoylamino)propionyl)-GLP-2(1-33);
L17K(3-(nonanoylamino)propionyl)-GLP-2(1-33);
L17K(3-(decanoylamino)propionyl)-GLP-2(1-33);
L17K(3-(undecanoylamino)propionyl)-GLP-2(1-33);
L17K(3-(dodecanoylamino)propionyl)-GLP-2(1-33);
L17K(3-(tridecanoylamino)propionyl)-GLP-2(1-33);
L17K(3-(tetradecanoylamino)propionyl)-GLP-2(1-33);
L17K(3-(pentadecanoylamino)propionyl)-GLP-2(1-33);
L17K(3-(hexadecanoylamino)propionyl)-GLP-2(1-33);
L17K(3-(heptadecanoylamino)propionyl)-GLP-2(1-33);
L17K(3-(octadecanoylamino)propionyl)-GLP-2(1-33);
L17K(3-(nonadecanoylamino)propionyl)-GLP-2(1-33);
L17K(3-(eicosanoylamino)propionyl)-GLP-2(1-33);
L17K((S)-4-carboxy-4-(octanoylamino)butanoyl)-GLP-2(1-33);
L17K((S)-4-carboxy-4-(nonanoylamino)butanoyl)-GLP-2(1-33);
L17K((S)-4-carboxy-4-(decanoylamino)butanoyl)-GLP-2(1-33);
L17K((S)-4-carboxy-4-(undecanoylamino)butanoyl)-GLP-2(1-33);
L17K((S)-4-carboxy-4-(dodecanoylamino)butanoyl)-GLP-2(1-33);
L17K((S)-4-carboxy-4-(tridecanoylamino)butanoyl)-GLP-2(1-33);
L17K((S)-4-carboxy-4-(tetradecanoylamino)butanoyl)-GLP-2(1-33);
L17K((S)-4-carboxy-4-(pentadecanoylamino)butanoyl)-GLP-2(1-33);
L17K((S)-4-carboxy-4-(hexadecanoylamino)butanoyl)-GLP-2(1-33);
L17K((S)-4-carboxy-4-(heptadecanoylamino)butanoyl)-GLP-2(1-33);
L17K((S)-4-carboxy-4-(octadecanoylamino)butanoyl)-GLP-2(1-33);
L17K((S)-4-carboxy-4-(nonadecanoylamino)butanoyl)-GLP-2(9-33);
L17K((S)-4-carboxy-4-(eicosanoylamino)butanoyl)-GLP-2(1-33);
L17K(4-(octanoylamino)butanoyl)-GLP-2(1-33);
L17K(4-(nonanoylamino)butanoyl)-GLP-2(1-33);
L17K(4-(decanoylamino)butanoyl)-GLP-2(1-33);
L17K(4-(undecanoylamino)butanoyl)-GLP-2(1-33);.
L17K(4-(dodecanoylamino)butanoyl)-GLP-2(1-33);
L17K(4-(tridecanoylamino)butanoyl)-GLP-2(1-33);
L17K(4-(tetradecanoylamino)butanoyl)-GLP-2(1-33);
L17K(4-(pentadecanoylamino)butanoyl)-GLP-2(1-33);
L17K(4-(hexadecanoylamino)butanoyl)-GLP-2(1-33);
L17K(4-(heptadecanoylamino)butanoyl)-GLP-2(1-33);
L17K(4-(octadecanoylamino)butanoyl)-GLP-2(1-33);
L17K(4-(nonadecanoylamino)butanoyl)-GLP-2(1-33);
L17K(4-(eicosanoylamino)butanoyl)-GLP-2(1-33);
A18K(3-(hexadecanoylamino)propionyl)-GLP-2(1-33);
D21K(3-(hexadecanoylamino)propionyl)-GLP-2(1-33);
N24K(3-(hexadecanoylamino)propionyl)-GLP-2(1-33);
Q28K(3-(hexadecanoylamino)propionyl)-GLP-2(1-33);
S5K(3-(hexadecanoytamino)propiony()/K30R-GLP-2(1-33);
S7K(3-(hexadecanoylamino)propionyl)/K30R-GLP-2(1-33);
D8K(3-(hexadecanoylamino)propionyl)/K30R-GLP-2(1-33);
E9K(3-(hexadecanoylamino)propionyl)/K30R-GLP-2(1-33);
M10K(3-(hexadecanoylamino)propionyl)/K30R-GLP-2(1-33);
N11K(3-(hexadecanoylamino)propionyl)/K30R-GLP-2(1-33);
T12K(3-(hexadecanoylamino)propionyl)/K30R-GLP-2(1-33);
I13K(3-(hexadecanoylamino)propionyl)/K30R-GLP-2(1-33);
L14K(3-(hexadecanoylamino)propionyl)/K30R-GLP-2(1-33);
D15K(3-(hexadecanoylamino)propionyl)/K30R-GLP-2(1-33);
N16K(3-(hexadecanoylamino)propionyl)/K30R-GLP-2(1-33);
L17K(3-(octanoylamino)propionyl)/K30R-GLP-2(1-33);
L17K(3-(nonanoylamino)propionyl)/K30R-GLP-2(1-33);
L17K(3-(decanoylamino)propionyl)/K30R-GLP-2(1-33);
L17K(3-(undecanoylamino)propionyl)/K30R-GLP-2(1-33);
L17K(3-(dodecanoylamino)propionyl)/K30R-GLP-2(1-33);
L17K(3-(tridecanoylamino)propionyl)/K30R-GLP-2(1-33);
L17K(3-(tetradecanoylamino)propionyl)/K30R-GLP-2(1-33);
L17K(3-(pentadecanoylamino)propionyl)/K30R-GLP-2(1-33);
L17K(3-(hexadecanoylamino)propionyl)/K30R-GLP-2(1-33);
L17K(3-(heptadecanoylamino)propionyl)/K30R-GLP-2(1-33);
L17K(3-(octadecanoylamino)propionyl)/K30R-GLP-2(1-33);
L17K(3-(nonadecanoylamino)propionyl)/K30R-GLP-2(1-33);
L17K(3-(eicosanoylamino)propionyl)/K30R-GLP-2(1-33);
L17K((S)-4-carboxy-4-(octanoylamino)butanoyl)/K30R-GLP-2(1-33);
L17K((S)-4-carboxy-4-(nonanoylamino)butanoyl)/K30R-GLP-2(1-33);
L17K((S)-4-carboxy-4-(decanoylamino)butanoyl)/K30R-GLP-2(1-33);
L17K((S)-4-carboxy-4-(undecanoylamino)butanoyl)/K30R-GLP-2(1-33);.
L17K((S)-4-carboxy-4-(dodecanoylamino)butanoyl)/K30R-GLP-2(1-33);
L17K((S)-4-carboxy-4-(tridecanoylamino)butanoyl)/K30R-GLP-2(1-33);
L17K((S)-4-carboxy-4-(tetradecanoylamino)butanoyl)/K30R-GLP-2(1-33);
L17K((S)-4-carboxy-4-(pentadecanoylamino)butanoyl)/K30R-GLP-2(1-33);
L17K((S)-4-carboxy-4-(hexadecanoylamino)butanoyl)/K30R-GLP-2(1-33);
L17K((S)-4-carboxy-4-(heptadecanoylamino)butanoyl)/K30R-GLP-2(1-33);
L17K((S)-4-carboxy-4-(octadecanoylamino)butanoyl)/K30R-GLP-2(1-33);
L17K((S)-4-carboxy-4-(nonadecanoylamino)butanoyl)/K30R-GLP-2(1-33);
L17K((S)-4-carboxy-4-(eicosanoylamino)butanoyl)/K30R-GLP-2(1-33);
L17K(4-(octanoylamino)butanoyl)/K30R-GLP-2(1-33);
L17K(4-(nonanoylamino)butanoyl)/K30R-GLP-2(1-33);
L17K(4-(decanoylamino)butanoyl)/K30R-GLP-2(1-33);
L17K(4-(undecanoylamino)butanoyl)/K30R-GLP-2(1-33);
L17K(4-(dodecanoylamino)butanoyl)/K30R-GLP-2(1-33);
L17K(4-(tridecanoylamino)butanoyl)/K30R-GLP-2(1-33);
L17K(4-(tetradecanoylamino)butanoyl)/K30R-GLP-2(1-33);
L17K(4-(pentadecanoylamino)butanoyl)/K30R-GLP-2(1-33);
L17K(4-(hexadecanoylamino)butanoyl)/K30R-GLP-2(1-33);
L17K(4-(heptadecanoylamino)butanoyl)/K30R-GLP-2(1-33);
L17K(4-(octadecanoylamino)butanoyl)/K30R-GLP-2(1-33);
L17K(4-(nonadecanoylamino)butanoyl)/K30R-GLP-2(1-33);
L17K(4-(eicosanoylamino)butanoyl)/K30R-GLP-2(1-33);
A18K(3-(hexadecanoylamino)propionyl)/K30R-GLP-2(1-33);
D21 K(3-(hexadecanoylamino)propionyl)/K30R-GLP-2(1-33);
N24K(3-(hexadecanoylamino)propionyl)/K30R-GLP-2(1-33);
Q28K(3-(hexadecanoylamino)propionyl)/K30R-GLP-2(1-33);
D3E/S5K(3-(hexadecanoylamino)propionyl)/K30R/D33E-GLP-2(1-33);
D3E/S7K(3-(hexadecanoy)amino)propionyl)/K30R/D33E-GLP-2(1-33);
D3E/D8K(3-(hexadecanoylamino)propionyl)/K30R/D33E-GLP-2(1-33);
D3E/E9K(3-(hexadecanoylamino)propionyl)/K30R/D33E-GLP-2(1-33);
D3E/M10K(3-(hexadecanoylamino)propionyl)/K30R/D33E-GLP-2(1-33);
D3E/N11K(3-(hexadecanoylamino)propionyl)/K30R/D33E-GLP-2(1-33);
D3E/T12K(3-(hexadecanoylamino)propionyl)/K30R/D33E-GLP-2(1-33);
D3E/I13K(3-(hexadecanoylamino)propionyl)/K30R/D33E-GLP-2(1-33);
D3E/L14K(3-(hexadecanoylamino)propionyl)/K30R/D33E-GLP-2(1-33);
D3E/D15K(3-(hexadecanoylamino)propionyl)/K30R/D33E-GLP-2(1-33);
D3E/N16K(3-(hexadecanoylamino)propionyl)/K30R/D33E-GLP-2(1-33);
D3E/L17K(3-(octanoylamino)propionyl)/K30R/D33E-GLP-2(1-33);
D3E/L17K(3-(nonanoylamino)propionyl)/K30R/D33E-GLP-2(1-33);
D3E/L17K(3-(decanoylamino)propionyl)/K30R/D33E-GLP-2(1-33);
D3E/L17K(3-(undecanoylamino)propionyl)/K30R/D33E-GLP-2(1-33);
D3E/L17K(3-(dodecanoylamino)propionyl)/K30R/D33E-GLP-2(1-33);
D3E/L17K(3-(tridecanoylamino)propionyl)/K30R/D33E-GLP-2(1-33);
D3E/L17K(3-(tetradecanoylamino)propionyl)/K30R/D33E-GLP-2(1-33);

D3E/L17K(3-(pentadecanoylamino)propionyl)/K30R/D33E-GLP-2(1-33);
D3E/L17K(3-(hexadecanoylamino)propionyl)/K30R/D33E-GLP-2(1-33);
D3E/L17K(3-(heptadecanoylamino)propionyl)/K30R/D33E-GLP-2(1-33);
D3E/L17K(3-(octadecanoy)amino)propionyl)/K30R/D33E-GLP-2(1-33);
D3E/L17K(3-(nonadecanoylamino)propionyl)/K30R/D33E-GLP-2(1-33);
D3E/L17K(3-(eicosanoylamino)propionyl)/K30R/D33E-GLP-2(1-33);
D3E/L17K((S)-4-carboxy-4-(octanoylamino)butanoyl)/K30R/D33E-GLP-2(1-33);
D3E/L17K((S)-4-carboxy-4-(nonanoylamino)butanoyl)/K30R/D33E-GLP-2(1-33);
D3E/L17K((S)-4-carboxy-4-(decanoylamino)butanoyl)/K30R/D33E-GLP-2(1-33);
D3E/L17K((S)-4-carboxy-4-(undecanoylamino)butanoyl)/K30R/D33E-GLP-2(1-33);
D3E/L17K((S)-4-carboxy-4-(dodecanoylamino)butanoyl)/K30R/D33E-GLP-2(1-33);
D3E/L17K((S)-4-carboxy-4-(tridecanoylamino)butanoyl)/K30R/D33E-GLP-2(1-33);
D3E/L17K((S)-4-carboxy-4-(tetradecanoylamino)butanoyl)/K30R/D33E-GLP-2(1-33);
D3E/L17K((S)-4-carboxy-4-(pentadecanoylamino)butanoyl)/K30R/D33E-GLP-2(1-33);
D3E/L17K((S)-4-carboxy-4-(hexadecanoylamino)butanoyl)/K30R/D33E-GLP-2(1-33);
D3E/L17K((S)-4-carboxy-4-(heptadecanoylamino)butanoyl)/K30R/D33E-GLP-2(1-33);
D3E/L17K((S)-4-carboxy-4-(octadecanoylamino)butanoyl)/K30R/D33E-GLP-2(1-33);
D3E/L17K((S)-4-carboxy-4-(nonadecanoylamino)butanoyl)/K30R/D33E-GLP-2(1-33);
D3E/L17K((S)-4-carboxy-4-(eicosanoylamino)butanoyl)/K30R/D33E-GLP-2(1-33);
D3E/L17K(4-(octanoylamino)butanoyl)/K30R/D33E-GLP-2(1-33);
D3E/L17K(4-(nonanoylamino)butanoyl)/K30R/D33E-GLP-2(1-33);
D3E/L17K(4-(decanoylamino)butanoyl)/K30RlD33E-GLP-2(1-33);
D3E/L17K(4-(undecanoylamino)butanoyl)/K30R/D33E-GLP-2(1-33);
D3E/L17K(4-(dodecanoylamino)butanoyl)/K30R/D33E-GLP-2(1-33);
D3E/L17K(4-(tridecanoy)amino)butanoyl)/K30R/D33E-GLP-2(1-33);
D3E/L17K(4-(tetradecanoylamino)butanoyl)/K30R/D33E-GLP-2(1-33);
D3E/L17K(4-(pentadecanoylamino)butanoyl)/K30R/D33E-GLP-2(1-33);
D3E/L17K(4-(hexadecanoylamino)butanoyl)/K30R/D33E-GLP-2(1-33);
D3E/L17K(4-(heptadecanoylamino)butanoyl)/K30R/D33E-GLP-2(1-33);
D3E/L17K(4-(octadecanoylamino)butanoyl)/K30R/D33E-GLP-2(1-33);
D3E/L17K(4-(nonadecanoylamino)butanoyl)/K30R/D33E-GLP-2(1-33);
D3E/L17K(4-(eicosanoylamino)butanoyl)/K30R/D33E-GLP-2(1-33);
D3E/A18K(3-(hexadecanoylamino)propionyl)/K30R/D33E-GLP-2(1-33);
D3E/D21K(3-(hexadecanoylamino)propionyl)/K30R/D33E-GLP-2(1-33);
D3E/N24K(3-(hexadecanoylamino)propionyl)/K30R/D33E-GLP-2(1-33); and
D3E/Q28K(3-(hexadecanoylamino)propionyl)/K30R/D33E-GLP-2(1-33).

Methods for the preparation of GLP-2, analogs thereof as well as GLP-2 derivatives can be found in e.g. WO 99/43361 and WO 00/55119.

In a further embodiment of the invention the glucagon-like peptide is an insulinotropic analog of exendin-4(1-39), e.g. Ser²Asp³-exendin-4(1-39) wherein the amino acid residues in position 2 and 3 have been replaced with serine and aspartic acid, respectively (this particular analog also being known in the art as exendin-3).

In a further embodiment of the invention the glucagon-like peptide is an exendin-4 derivative wherein the substituent introduced is selected from amides, carbohydrates, alkyl groups, esters and lipophilic substituents. An example of an insulinotropic derivatives of exendin-4(1-39) and analogs thereof is Tyr³¹-exendin-4(1-31)-amide.

In another embodiment of the invention the glucagon-like peptide is a stable exendin-4 peptide. In another embodiment of the invention the glucagon-like peptide is a DPP-IV protected exendin-4 peptide. In another embodiment of the invention the glucagon-like peptide is an immunomodulated exendin-4 peptide.

Methods for the preparation of exendin-4, analogs thereof as well as exendin-4 derivatives can be found in e.g. WO 99/43708, WO 00/41546 and WO 00/55119.

The parent glucagon-like peptide can be produced by peptide synthesis, e.g. solid phase peptide synthesis using Boc- or Fmoc-chemistry or other well established techniques. The parent glucagon-like peptide can also be produced by a method which comprises culturing a host cell containing a DNA sequence encoding the polypeptide and capable of expressing the polypeptide in a suitable nutrient medium under conditions permitting the expression of the peptide, after which the resulting peptide is recovered from the culture.

The medium used to culture the cells may be any conventional medium suitable for growing the host cells, such as minimal or complex media containing appropriate supplements. Suitable media are available from commercial suppliers or may be prepared according to published recipes (*e.g.* in catalogues of the American Type Culture Collection). The peptide produced by the cells may then be recovered from the culture medium by conventional procedures including separating the host cells from the medium by centrifugation or filtration, precipitating the proteinaceous components of the supernatant or filtrate by means of a salt, *e.g.* ammonium sulphate, purification by a variety of chromatographic procedures, *e.g.* ion exchange chromatography, gel filtration chromatography, affinity chromatography, or the like, dependent on the type of peptide in question.

The DNA sequence encoding the parent peptide may suitably be of genomic or cDNA origin, for instance obtained by preparing a genomic or cDNA library and screening for DNA sequences coding for all or part of the peptide by hybridisation using synthetic oligonucleotide probes in accordance with standard techniques (see, for example, Sambrook, J, Fritsch, EF and Maniatis, T, Molecular Cloning: A Laboratory Manual, Cold Spring Harbor Laboratory Press, New York, 1989). The DNA sequence encoding the peptide may also be prepared synthetically by established standard methods, e.g. the phosphoamidite method described by Beaucage and Caruthers, Tetrahedron Letters 22 (1981), 1859 -1869, or the method described by Matthes et al., EMBO Journal 3 (1984), 801 - 805. The DNA sequence may also be prepared by polymerase chain reaction using specific primers, for instance as described in US 4,683,202 or Saiki et al., Science 239 (1988), 487 - 491.

The DNA sequence may be inserted into any vector which may conveniently be subjected to recombinant DNA procedures, and the choice of vector will often depend on the host cell into which it is to be introduced. Thus, the vector may be an autonomously replicating vector, *i.e.* a vector which exists as an extrachromosomal entity, the replication of which is independent of chromosomal replication, *e.g.* a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the peptide is operably linked to additional segments required for transcription of the DNA, such as a promoter. The promoter may be any DNA sequence which shows transcriptional activity in the host cell of choice and may be derived from genes encoding proteins either homologous or heterologous to the host cell. Examples of suitable promoters for directing the transcription of the DNA encoding the peptide of the invention in a variety of host cells are well known in the art, cf. for instance Sambrook *et al., supra.*

The DNA sequence encoding the peptide may also, if necessary, be operably connected to a suitable terminator, polyadenylation signals, transcriptional enhancer sequences, and translational enhancer sequences. The recombinant vector of the invention may further comprise a DNA sequence enabling the vector to replicate in the host cell in question.

The vector may also comprise a selectable marker, *e.g.* a gene the product of which complements a defect in the host cell or one which confers resistance to a drug, *e.g.* ampicillin, kanamycin, tetracyclin, chloramphenicol, neomycin, hygromycin or methotrexate.

To direct a parent peptide of the present invention into the secretory pathway of the host cells, a secretory signal sequence (also known as a leader sequence, prepro sequence or pre sequence) may be provided in the recombinant vector. The secretory signal sequence is joined to the DNA sequence encoding the peptide in the correct reading frame. Secretory signal sequences are commonly positioned 5' to the DNA sequence encoding the peptide. The secretory signal sequence may be that normally associated with the peptide or may be from a gene encoding another secreted protein.

The procedures used to ligate the DNA sequences coding for the present peptide, the promoter and optionally the terminator and/or secretory signal sequence, respectively, and to insert them into suitable vectors containing the information necessary for replication, are well known to persons skilled in the art (cf., for instance, Sambrook *et al.., supra*).

The host cell into which the DNA sequence or the recombinant vector is introduced may be any cell which is capable of producing the present peptide and includes bacteria, yeast, fungi and higher eukaryotic cells. Examples of suitable host cells well known and used in the art are, without limitation, *E. coli, Saccharomyces cerevisiae,* or mammalian BHK or CHO cell lines.

Pharmaceutical compositions containing a glucagon-like peptide purified according to the present invention typically contain various pharmaceutical excipients, such as preservatives, isotonic agents and surfactants. The preparation of pharmaceutical compositions is well-known to the skilled person. For convenience reference is made to Remington: The Science and Practice of Pharmacy, 19th edition, 1995.

Pharmaceutical compositions containing a glucagon-like peptide purified according to the present invention may be administered parenterally to patients in need of such treatment. Parenteral administration may be performed by subcutaneous injection, intramuscular injection, or intraveneous injection by means of a syringe, optionally a pen-like syringe. Alternatively administration can be performed by infusion, e.g. by use of an infusion pump.

The present invention is further illustrated by the following examples which, however, are not to be construed as limiting the scope of protection. The features disclosed in the foregoing description and in the following examples may, both separately and in any combination thereof, be material for realising the invention in diverse forms thereof.

### EXAMPLES

### Example 1

**Analytical RP-HPLC**. RP-HPLC analysis for identification/verification of collected peaks was carried out on a Waters Symmetry RP-18, 3.5 µm, 100Å, 4.6 x 150 mm column. Buffer A consisted of 0.15 M (NH₄)₂SO₄ in 7.8 % (w/w) acetonitrile, pH 2.5, and buffer B contained 63.4 % (w/w) acetonitrile. Linear gradients from 37-44.1% B in 15 min followed by 44.1-100 % B in 10 min were run at a flow rate of 1 ml/min. The chromatographic temperature was kept at 60°C and UV detection was performed at 214 nm.

### Example 2

Arg³⁴GLP-1₍₇₋₃₇₎ was expressed in yeast (*S. cerevisiae*) by conventional recombinant DNA technology, e.g. as described in WO 98/08871. Arg³⁴GLP-1 ₍₇₋₃₇₎ in the fermentation broth was then purified by conventional reversed phase chromatography and subsequently precipitated at the isoelectric pH of the peptide, i.e. at pH 5.4. The precipitate was isolated by centrifugation.

The isoelectric precipitate containing Arg³⁴GLP-1₍₇₋₃₇₎ and related impurities, among others the truncated impurity Arg³⁴GLP-1₍₉₋₃₇₎, was dissolved in water and pH was adjusted to 3.5. 15 mL of the solution (0.91 mg/mL) was loaded to a 20 mL 120Å C₄-substituted (dimethylbutyl dimethylsilyl) silica resin (particle size 10 µm, YMC) equilibrated with 40 mL 0.15 mol/kg ammoniumsulfate, 5 mmol/kg citric acid, 25% (w/w) ethanol pH 3.5. The column was washed with 10 mL equilibration solution and elution was performed with a linear gradient of 35-45% ethanol (0.15 mol/kg ammoniumsulfate, 5 mmol/kg citric acid) during 240 mL.

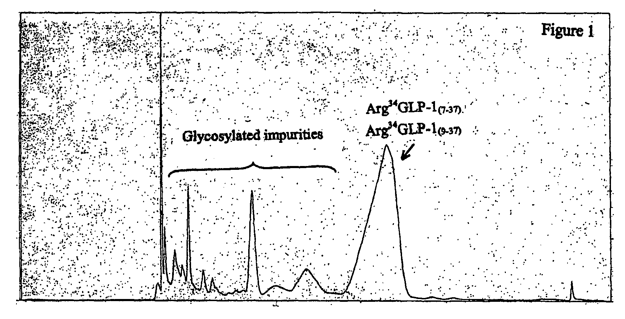

A chromatogram of the preparative purification is shown in Fig. 1. From the chromatographic profile it can be observed that glycosylated impurities were separated, however, no distinct peaks nor separation between the truncated form and the target GLP-1 moiety were obtained. Neither was any separation between Arg³⁴GLP-1 ₍₇₋₃₇₎ and Arg³⁴GLP-1 ₍₉₋₃₇₎ observed by RP-HPLC analysis.

### Example 3

Arg³⁴GLP-1₍₇₋₃₇₎ was expressed in yeast, captured by RP-LC and precipitated as described in example 2.

The isoelectric precipitate containing Arg³⁴GLP-1₍₇₋₃₇₎ and related impurities, among others the truncated impurity Arg³⁴GLP-1₍₉₋₃₇₎, was dissolved in water and pH was adjusted to 7.5. 15 mL of the solution (0.91 mg/mL) was loaded to a 20 mL 120Å C₄ substituted (dimethylbutyl dimethylsilyl) silica gel (particle size 10 µm, YMC) equilibrated with 40 mL 5 mmol/kg sodium dihydrogen phosphate, 210 mmol/kg potassium acetate, 25% (w/w) ethanol pH 7.5. The column was washed with 10 mL equilibration solution and elution was performed with a linear gradient of 30-40% ethanol (5 mmol/kg sodium dihydrogen phosphate, 210 mmol/kg potassium acetate) during 240 mL

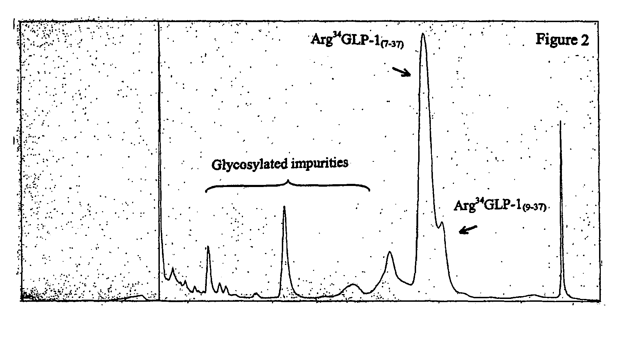

A chromatogram of the preparative purification is shown in Fig. 2. Solely from the chromatographic profile it can be observed that glycosylated impurities were separated and furthermore, separation between the truncated form and the target GLP-1 moiety was obtained at a buffer controlled pH of 7.5 of the chromatographic solvents. In addition, RP-HPLC analysis results of fractions given in Table 1 show that the content of the truncated form, Arg³⁴GLP-1₍₉₋₃₇), in the main peak has been reduced to an acceptable level.

**Table 1. RP-HPLC analysis of example 3. The analysis was performed as described in example 1.**

| | Arg³⁴GLP-1₍₇₋₃₇₎ content | Arg³⁴GLP-1₍₉₋₃₇₎ content |
|---|---|---|
| Sample for loading | 55% | 11% |
| Main peak | 92% | 5% |
| Impurity peak | 10% | 74% |

By comparing chromatographic profiles of examples 1 and 2, an additional advantage of neutral pH is noticed: a much higher and narrower main peak and thus a desired higher pool concentration is obtained. Insignificant differences in set up between examples 1 and 2 are: different buffer system to control pH of the respective chromatographic runs, and different salt systems. Further, the same gradient steepness was employed, but with different ethanol starting concentration to obtain similar retention.

### Example 4

Arg³⁴GLP-1₍₇₋₃₇₎ was expressed in yeast (*S. cerevisiae*) by conventional recombinant DNA technology, e.g. as described in WO 98/08871. Arg³⁴GLP-1₍₇₋₃₇₎ in the cell free fermentation broth was purified by cation exchange chromatography and pH of the resulting pool containing Arg³⁴GLP-1₍₇₋₃₇₎ was adjusted to pH 9.0. 10 mL of the pool containing Arg³⁴GLP-1₍₇₋₃₇₎ (3.49 mg/mL) and related impurities, among others the truncated impurity Arg³⁴GLP-1₍₉₋₃₇₎, was loaded to a 20 mL 200Å C₁₈ substituted (octadecyl dimethyl silyl) silica gel (particle size 15µm) equilibrated with 40 mL of a solvent containing 0.15 mol/kg ammoniumsulfate, 5 mmol/kg citric acid, 25% (w/w) ethanol, pH 3.5. The column was washed with 10 mL equilibration solution and elution was performed with a linear gradient of 35-45% ethanol (0.15 mol/kg ammoniumsulfate, 5 mmol/kg citric acid) during 240 mL. The temperature was kept at 23°C during the entire run.

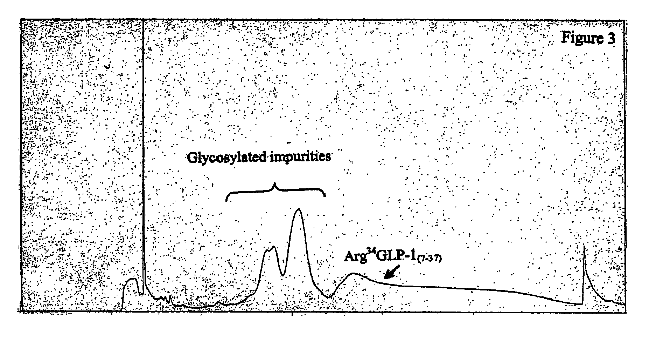

A chromatogram of the preparative purification is shown in Fig. 3. Glycosylated impurities were separated, however, the target GLP-1 moiety was not eluted properly because it fibrillated on the column and was not possible to collect at all. Thus, low pH in connection with a highly hydrophobic ligate, such as C₁₈, should not be employed.

### Example 5

Arg³⁴GLP-1₍₇₋₃₇₎ was expressed in yeast (*S*. *cerevisiae*) by conventional recombinant DNA technology, e.g. as described in WO 98/08871. Arg³⁴GLP-1₍₇₋₃₇₎ was captured by cation exchange chromatography as described in example 4.

10 mL of the pool (pH 8.9 at room temperature) containing Arg³⁴GLP-1₍₇₋₃₇₎ (3.49 mg/mL) and related impurities, among others the truncated impurity Arg³⁴GLP-1₍₉₋₃₇₎, was loaded to a 20 mL 200Å C₁₈ substituted (octadecyl dimethyl silyl) silica gel (particle size 15µm) equilibrated with 40 mL of a solvent containing 250 mmol/kg potassium chloride, 5 mmol/kg potassium dihydrogen phosphate, 25%(w/w) ethanol, pH 7.5. The column was washed with 10 mL equilibration solution and elution was performed with a linear gradient of 30-40% ethanol (250 mmol/kg potassium chloride, 5 mmol/kg potassium dihydrogen phosphate) during 240 mL. The temperature was kept at 23°C during the entire run.

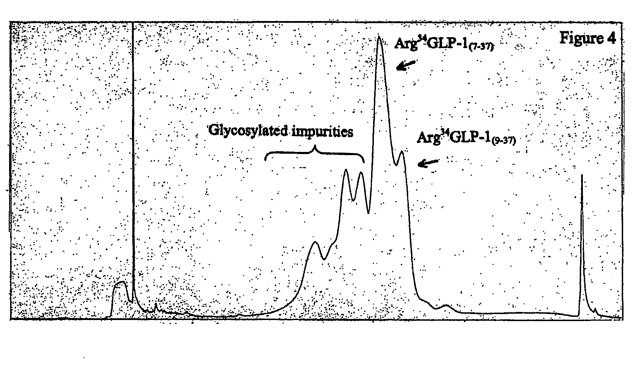

A chromatogram of the preparative purification is shown in Fig. 4. Solely from the chromatographic profile it can be observed that glycosylated impurities were separated and furthermore, separation between the truncated form and the target GLP-1 moiety was obtained at a buffer controlled pH of 7.5 of the chromatographic solvents.

Comparing chromatographic profiles of examples 4 and 5 it is shown that the target GLP-1 moiety may only be collected from the chromatographic run at neutral pH. Insignificant differences in set up between examples 4 and 5 are: different buffer system to control pH of the respective chromatographic runs, and different salt systems. Further, the same gradient steepness was employed, but with different ethanol starting concentration to obtain similar retention.

### Example 6

Arg³⁴GLP-1₍₇₋₃₇₎ was expressed in yeast and captured by cation exchange chromatography as described in example 4.

51 mL of the pool (pH 7.45 at 22.5°C) containing Arg³⁴GLP-1₍₇₋₃₇₎ (0.7 mg/mL) and related impurities, among others the truncated impurity Arg³⁴GLP-1₍₉₋₃₇₎, was loaded to a 20 mL 200Å C₁₈ substituted (octadecyl dimethyl silyl) silica gel (particle size 15µm) equilibrated with 40 mL of a solvent containing 250 mmol/kg potassium chloride, 5 mmol/kg potassium dihydrogen phosphate, 25%(w/w) ethanol, pH 7.5. The column was washed with 10 mL equilibration solution and elution was performed with a linear gradient of 30-40% ethanol (250 mmol/kg potassium chloride, 5 mmol/kg potassium dihydrogen phosphate) during 240 mL. The temperature was kept at 4°C during the entire run.

Distinct peaks and separation between Arg³⁴GLP-1₍₇₋₃₇₎, the truncated form Arg³⁴GLP-1₍₉₋₃₇₎ and glycosylated forms of the peptide at this temperature were obtained, similar to that presented in example 5. The insignificant difference in set up between this example and example 5 is the use of a different sample for loading.

### Example 7

Arg³⁴GLP-1₍₇₋₃₇₎ was expressed in yeast and captured by cation exchange chromatography as described in example 4.

51 mL of the pool (pH 8.88 at 24.6°C) containing Arg³⁴GLP-1₍₇₋₃₇₎ (0.7 mg/mL) and related impurities, among others the truncated impurity Arg³⁴GLP-1₍₉₋₃₇₎, was loaded to a 20 mL 200Å C₁₈ substituted (octadecyl dimethyl silyl) silica gel (particle size 15µm) equilibrated with 40 mL of a solvent containing 250 mmol/kg potassium chloride, 5 mmol/kg potassium dihydrogen phosphate, 25%(w/w) ethanol, pH 7.5. The column was washed with 10 mL equilibration solution and elution was performed with a linear gradient of 25-35% ethanol (250 mmol/kg potassium chloride, 5 mmol/kg potassium dihydrogen phosphate) during 240 mL. The temperature was kept at 50°C during the entire run.

Distinct peaks and separation between Arg³⁴GLP-1₍₇₋₃₇₎, the truncated form Arg³⁴GLP-1₍₉₋₃₇₎ and glycosylated forms of the peptide at this temperature were obtained, similar to that presented in example 5. The insignificant difference in set up between this example and example 5 is the use of a different sample for loading.

### Example 8

Arg³⁴GLP-1₍₇₋₃₇₎ was expressed in yeast and captured by cation exchange chromatography as described in example 4.

51 mL of the pool (pH 8.89 at 20.9°C) containing Arg³⁴GLP-1₍₇₋₃₇₎ (0.7 mg/mL) and related impurities, among others the truncated impurity Arg³⁴GLP-1₍₉₋₃₇₎, was loaded to a 20 mL 120Å C₁₈ substituted (octadecyl dimethyl silyl) silica gel (particle size 15µm) equilibrated with 40 mL of a solvent containing 250 mmol/kg potassium chloride, 5 mmol/kg potassium dihydrogen phosphate, 25%(w/w) ethanol, pH 7.5. The column was washed with 10 mL equilibration solution and elution was performed with a linear gradient of 30-40% ethanol (250 mmol/kg potassium chloride, 5 mmol/kg potassium dihydrogen phosphate) during 240 mL. The temperature was kept at 23°C during the entire run.

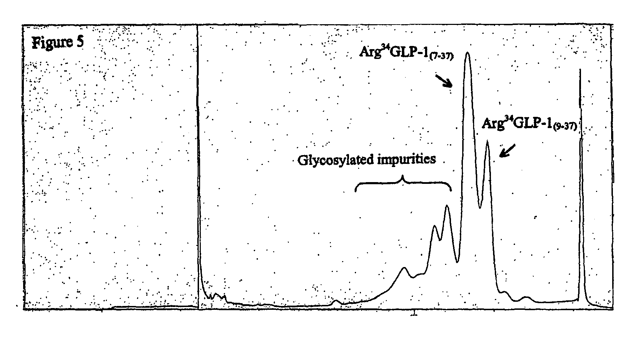

A chromatogram of the preparative purification is shown in Fig. 5. Solely from the chromatographic profile it can be observed that glycosylated impurities were separated and further more, separation between the truncated form and the target GLP-1 moiety was obtained at a buffer controlled pH of 7.5 of the chromatographic solvents. In fact, a higher resolution between the Arg³⁴GLP-1₍₇₋₃₇₎ peak and the surrounding peaks including Arg³⁴GLP-1₍₉₋₃₇₎ was achieved with the 120Å material than with the 200Å material described in example 5. The insignificant difference in set up between this example and example 5 is the use of a different sample for loading.

### Example 9

Arg³⁴GLP-1₍₇₋₃₇₎ was expressed in yeast and captured by cation exchange chromatography as described in example 4.

63 mL of the pool (pH 8.84 at 22.1°C) containing Arg³⁴GLP-1₍₇₋₃₇₎ (0.6 mg/mL) and related impurities, among others the truncated impurity Arg³⁴GLP-1₍₉₋₃₇₎, was loaded to a 20 mL 120Å C₁₈ substituted (octadecyl dimethyl silyl) silica gel (particle size 15µm) equilibrated with 40 mL of a solvent containing 250 mmol/kg potassium chloride, 5 mmol/kg potassium dihydrogen phosphate, 25%(w/w) ethanol, pH 7.0. The column was washed with 10 mL equilibration solution and elution was performed with a linear gradient of 30-40% ethanol (250 mmol/kg potassium chloride, 5 mmol/kg potassium dihydrogen phosphate) during 240 mL. The temperature was kept at 23°C during the entire run.

Distinct peaks and separation between Arg³⁴GLP-1₍₇₋₃₇₎, the truncated form Arg³⁴GLP-1₍₉₋₃₇₎ and glycosylated forms of the peptide at this pH were obtained, similar to that presented in example 8. The insignificant difference in set up between this example and example 8 is the use of a different sample for loading.

### Example 10

Arg³⁴GLP-1₍₇₋₃₇₎ was expressed in yeast and captured by cation exchange chromatography as described in example 4.

Purification was performed as described in example 9, but pH of the solvents was 8.0.

Distinct peaks and separation between Arg³⁴GLP-1₍₇₋₃₇₎, the truncated form Arg³⁴GLP-1₍₉₋₃₇₎ and glycosylated forms of the peptide at this pH were obtained, similar to that presented in example 8. The insignificant difference in set up between this example and example 8 is the use of a different sample for loading.

### Example 11

Arg³⁴GLP-1₍₇₋₃₇₎ was expressed in yeast, captured by RP-LC and precipitated as described in example 2.

The isoelectric precipitate containing Arg³⁴GLP-1₍₇₋₃₇₎ and related impurities, among others the truncated impurity Arg³⁴GLP-1₍₉₋₃₇₎, was dissolved in water and pH was adjusted to 7.5. 15 mL of the solution (0.91 mg/mL) was loaded to a 20 mL 120Å C₄ substituted (dimethylbutyl dimethylsilyl) silica gel (particle size 10 µm, YMC) equilibrated with 40 mL 210 mmol/kg potassium acetate, 25% (w/w) ethanol pH 7.5. The column was washed with 10 mL equilibration solution and elution was performed with a linear gradient of 30-40% ethanol (210 mmol/kg potassium acetate) during 240 mL, that is, in a system without a buffer substance at the applied pH.

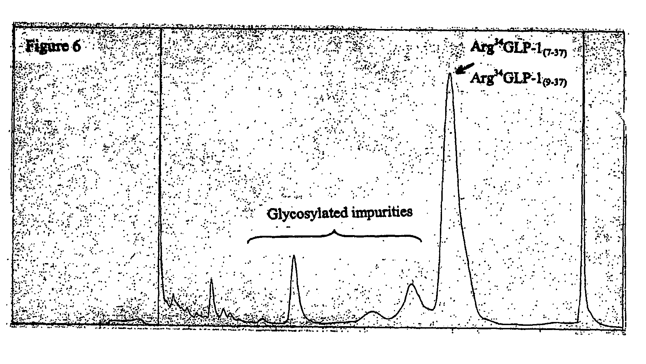

A chromatogram of the preparative purification is shown in Fig. 6. Solely, from the chromatographic profile it can be observed that glycosylated impurities were separated, however, no distinct peaks nor separation between the truncated form and the target GLP-1 moiety were obtained.

Comparing chromatographic profiles of examples 3 and 11 it is shown that the target GLP-1 moiety may be separated from the truncated impurity at pH 7.5 if pH is controlled by a buffer substance, and thus, running the separation at neutral pH without a buffer substance to control pH decreases the separation efficiency of the system. No other differences in set up between examples 3 and 11 were applied.

### Example 12

Arg³⁴GLP-1₍₇₋₃₇₎ was expressed in yeast (*S. cerevisiae*) by conventional recombinant technology as described elsewhere (WO 98/08871). Arg³⁴GLP-1₍₇₋₃₇₎ in the fermentation broth was then purified by reversed phase chromatography with elution in a glycine buffer at pH 9.0.

33 mL of the eluate at pH 7.5 (1.1 mg/mL) was loaded to a 20 mL Source 15 RPC (Amersham Pharmacia Biotech) polystyrene/divinyl benzene (particle size 15 µm) column, equilibrated with 40 mL 25% ethanol, 250 mmol/kg potassium chloride, 5 mmol/kg sodium citrate, pH 6.75. The column was washed with 10 mL equilibration solution and elution was performed with a linear gradient of 35 - 45% ethanol (250 mmol/kg potassium chloride, 5 mmol/kg sodium citrate), pH 6.75 during 240 mL. The temperature was kept at 23°C during the entire run.

Distinct peaks and separation between Arg³⁴GLP-1₍₇₋₃₇₎ and glycosylated forms of the peptide was obtained.

### Example 13

Arg³⁴GLP-1₍₇₋₃₇₎ was expressed in yeast (*S. cerevisiae*) by conventional recombinant technology as described elsewhere (WO 98/08871). Arg³⁴GLP-1₍₇₋₃₇₎ in the fermentation broth was then purified by reversed phase chromatography with elution in a glycine buffer at pH 9.0.

4.6 mL of the solution (1.2 mg/mL) was loaded to a 3 mL RPC PolyBio (BioSepra) (particle size 15 µm) column, equilibrated with 6 mL 25% ethanol, 250 mmol/kg potassium chloride, 5 mmol/kg NaH₂PO₄, pH 7.5. The column was washed with 1.5 mL equilibration solution and elution was performed with a linear gradient of 35 - 45% ethanol (250 mmol/kg potassium chloride, 5 mmol/kg NaH₂PO₄), pH 7.5 during 36 mL. The temperature was kept at 23°C during the entire run.

Distinct peaks and separation between Arg³⁴GLP-1₍₇₋₃₇₎ and glycosylated forms of the peptide was obtained.

### Example 14

Arg³⁴GLP-1₍₇₋₃₇₎ was expressed in yeast (*S*. *cerevisiae)* by conventional recombinant technology as described elsewhere (WO 98/08871). Arg³⁴GLP-1₍₇₋₃₇₎ in the fermentation broth was then purified by conventional reversed phase chromatography and subsequently precipitated at the isoelectric pH of the peptide, i.e. at pH 5.4. The precipitate was isolated by centrifugation.

30 g isoprecipitate was dissolved in 1.5 L water. pH was adjusted to 8.37. Pools of 220 mL of the solution were adjusted to approximately pH 3.5 and loaded to a 78 mL Source 30S (Amersham Pharmacia Biotech) column equilibrated with 45% (w/w) ethanol, 20 mmol/kg citric acid, 75 mol/kg potassium chloride pH 3.5. The column was washed with 160 mL 45% (w/w) ethanol, 20 mol/kg citric acid, 87.5 mol/kg potassium chloride, pH 3.5 and Arg³⁴GLP-1₍₇₋₃₇₎ was eluted with 400 mL 200 mmol/kg glycin, pH 9.0. Eluates were pooled. 160 mL of the CIEC-pool (1.8 mg/mL) was adjusted to pH 7.5 and loaded to a 78 mL 120Å C₁₈ substituted (OdDMeSi) silica gel (particle size 15µm) equilibrated with 160 mL of a solvent containing 250 mmol/kg sodium chloride, 5 mmol/kg sodium dihydrogen phosphate, 25%(w/w) ethanol, pH 7.0. The column was washed with 40 mL equilibration solution and elution was performed with a linear gradient of 28-38% ethanol (250 mmol/kg sodium chloride, 5 mmol/kg sodium dihydrogen phosphate) during 936 mL. The temperature was kept at 23°C during the entire run.

Distinct peaks and separation between Arg³⁴GLP-1₍₇₋₃₇₎ and glycosylated forms of the peptide were obtained.

Insignificant differences in set up between examples 14 and 5 are: higher load, different sample for loading, different salt- and buffer systems and larger scale.

### Example 15

Arg³⁴Lys²⁶N^{ε}(γ-Glu(N^{α}-hexadecanoyl))GLP-1₍₇₋₃₇₎ was prepared from the parent peptide, Arg³⁴GLP-1₍₇₋₃₇₎, by acylation as described in WO 00/55119.

Arg³⁴Lys²⁶N^{ε}(γ-Glu(N^{α}-hexadecanoyl))GLP-1₍₇₋₃₇₎ was loaded to a 20 mL C₁₈ substituted (octadecyl dimethyl silyl) silica gel (particle size 15µm) equilibrated with 40 mL 25% w/w ethanol. The column was washed with 10 mL 25% w/w ethanol, 250 mmol/kg potassium chloride, 20 mmol/kg Bis-tris propane, pH 6.5 and Arg³⁴Lys²⁶N^{ε}(γ-Glu(N^{α}-hexadecanoyl))GLP-1₍₇₋₃₇₎ was eluted with a linear gradient of 37-47.5% ethanol (250 mmol/kg potassium chloride, 20 mmol/kg Bis-tris propane, pH 6.5) during 480 mL. The temperature was kept at 50 °C during the entire run.

Distinct peaks and separation between Arg³⁴Lys²⁶N^{ε}(γ-Glu(N^{α}-hexadecanoyl))GLP-1₍₇₋₃₇₎ and un- and diacylated forms was obtained and furthermore, unidentified related impurities were separated in the rear flank.

### Example 16

Lys¹⁷Arg³⁰GLP-2₍₁₋₃₃₎ was expressed in yeast (*S. cerevisiae*) by conventional recombinant DNA technology, e.g. as described in WO 98/08871. Lys¹⁷Arg³⁰GLP-2₍₁₋₃₃₎ was captured by RP-LC and precipitated at the isoelectric pH of Lys¹⁷Arg³⁰GLP-2₍₁₋₃₃₎ (pH 4.0). The peptide was further purified on a hydroxyapatit column and eluted with 100 mmol/kg potassium hydrogen phosphate, pH 7.8. The capture pool was purified by anion exchange chromatography at pH 8.

The pool from the anion exchange step was loaded to a 4 L 100Å C₁₈ substituted (octadecyl dimethyl silyl) silica gel (particle size 15 µm) equilibrated with 25% w/w ethanol, 10 mmol/kg sodium dihydrogen phosphate, 250 mmol/kg potassium chloride, pH 7.5. The column was washed with 7.8 L 25% ethanol (10 mmol/kg sodium dihydrogen phosphate, 250 mmol/kg potassium chloride, pH 7.5) followed by 23.6 L 34% ethanol (10 mmol/kg sodium dihydrogen phosphate, 250 mmol/kg potassium chloride, pH 7.5). Elution of Lys¹⁷Arg³⁰GLP-2₍₁₋₃₃₎ was performed with a linear gradient of 34-40% ethanol (10 mmol/kg sodium dihydrogen phosphate, 250 mmol/kg potassium chloride, pH 7.5) during 78.6 L. The temperature was kept at 23°C during the entire run. Distinct peaks and separation between Lys¹⁷Arg³⁰GLP-2₍₁₋₃₃₎ and a met-oxidated form of the peptide was obtained. Furthermore, Lys¹⁷Arg³⁰GLP-2₍₁₋₃₃₎ was separated from the truncated impurity (des His-Ala Lys¹⁷Arg³⁰GLP-2₍₁₋₃₃₎.

### Example 17

Arg³⁰Lys¹⁷N^{ε}(β-Ala(N^{α}-hexadecanoyl)) GLP-2₍₁₋₃₃₎ was prepared from the parent peptide, Lys¹⁷Arg³⁰GLP-2₍₁₋₃₃₎, by acylation as described in WO 00/55119.

Arg³⁰Lys¹⁷N^{ε}(β-Ala(N^{α}-hexadecanoyl)) GLP-2₍₁₋₃₃₎ was loaded to a 4 L 100Å C₁₈ substituted (octadecyl dimethyl silyl) silica gel (particle size 15µm) equilibrated with 12 L 40% w/w ethanol, 10 mmol/kg sodium dihydrogen phosphate, 250 mmol/kg potassium chloride, pH 7.5. The column was washed with 4 L of the equilibration solvent and 4 L of 43% w/w ethanol, 10 mmol/kg sodium dihydrogen phosphate, 227 mmol/kg potassium chloride, pH 7.5. Arg³⁰Lys¹⁷N^{ε}(β-Ala(N^{α}-hexadecanoyl)) GLP-2₍₁₋₃₃₎ was eluted with a linear gradient of 45-60% w/w ethanol (211 - 94 mmol/kg potassium chloride, 10 mmol/kg sodium dihydrogen phosphate, pH 7.5.) during 120L. The temperature was kept at 23°C during the entire run.

Distinct peaks and separation between Arg³⁰Lys¹⁷N^{ε}(β-Ala(N^{α}-hexadecanoyl)) GLP-2₍₁₋₃₃₎ and the un-acylated form plus other related impurities was obtained.

### Example 18

Exendin-4(1-39) (with the amino acid sequence HGEGTFTSDLSKQMEEEAVRLFIEWLKNGGPSSGAPPPS) and Exendin-4(2-39) (with the amino acid sequence GEGTFTSDLSKQMEEEAVRLFIEWLKNGGPSSGAPPPS) were synthesized by standard solid phase synthesis methods using Fmoc chemistry.

A solution of Exendin-4(1-39) containing the related impurity Exendin-4(2-39) were dissolved in water to a total concentration of 1 mg peptide/mL. 6 mL of the solution was loaded to a 7.85 mL column containing 120 Å C18 substituted (octadecyl-dimethyl silyl) silica gel (particle size 15 µm) equilibrated with 15.7 mL of a solvent containing 25% w/w ethanol, 0.069 % w/w sodium di-hydrogen phosphate monohydrate, 2.06% w/w potassium acetate, pH 4.02. The column was washed with 3.9 mL equilibration solution. The elution was performed with a isocratic gradient of 36% ethanol during 157 mL (20 CV) followed by a 23.6 mL (3 CV) linear gradient from 36% to 39% ethanol in 0.069% w/w sodium di-hydrogen phosphate monohydrate, 2.06% w/w potassium acetate, pH 4.02. Subsequently, the elution was performed by a step gradient to 59% ethanol in 0.069% w/w sodium di-hydrogen phosphate monohydrate, 2.06% w/w potassium acetate, pH 4.02 maintained for 7.85 mL (1 CV). The experiment was performed at room temperature.

Distinct peaks and separation between Exendin-4(1-39) and Exendin-4(2-39) were obtained, Exendin-4(1-39) eluting before Exendin-4(2-39).

### Example 19

Exendin-4(1-39) and Exendin-4(2-39) were synthesized by standard solid phase synthesis methods using Fmoc chemistry.

A solution of Exendin-4(1-39) containing the related impurity Exendin-4(2-39) were dissolved in water to a total concentration of 1 mg peptide/mL. 8 mL of the solution was loaded to a 7.85 mL column containing 120 Å C18 substituted (octadecyl-dimethyl silyl) silica gel (particle size 15 µm) equilibrated with 15.7 mL of a solvent containing 25% w/w ethanol, 0.069 % w/w sodium di-hydrogen phosphate monohydrate, 2.06% w/w potassium acetate, pH 3.5. The column was washed with 3.9 mL equilibration solution. The elution was performed with a isocratic gradient of 37% ethanol during 110 mL (14 CV) followed by a 24 mL (3 CV) linear gradient from 37% to 39% ethanol in 0.069% w/w sodium di-hydrogen phosphate monohydrate, 2.06% w/w potassium acetate, pH 3.5. Subsequently, the elution was performed by a linear gradient to 59% ethanol in 0.069% w/w sodium di-hydrogen phosphate monohydrate, 2.06% w/w potassium acetate, pH 3.5 during 71 mL (9 CV). The experiment was performed at room temperature.

Distinct peaks and separation between Exendin-4(1-39) and Exendin-4(2-39) were obtained, Exendin-4(1-39) eluting before Exendin-4(2-39).

### Example 20

L-His¹ Exendin-4(1-39) and D-His¹ Exendin-4(1-39) (with the amino acid sequence HGEGTFTSDLSKQMEEEAVRLFIEWLKNGGPSSGAPPPS) were synthesized by standard solid phase synthesis methods using Fmoc chemistry.

A solution of L-His¹ Exendin-4(1-39) containing the related impurity D-His¹ Exendin-4(1-39) were dissolved in water to a total concentration of 1 mg peptide/mL. 8 mL of the solution was loaded to a 7.85 mL column containing 120 Å C18 substituted (octadecyl-dimethyl silyl) silica gel (particle size 15 µm) equilibrated with 15.7 mL of a solvent containing 25% w/w ethanol, 0.069 % w/w sodium di-hydrogen phosphate monohydrate, 2.06% w/w potassium acetate, pH 3.5. The column was washed with 3.9 mL equilibration solution. The elution was performed with a isocratic gradient of 37% ethanol during 63 mL (8 CV) followed by a 24 mL (3 CV) linear gradient from 37% to 39% ethanol in 0.069% w/w sodium di-hydrogen phosphate monohydrate, 2.06% w/w potassium acetate, pH 3.5. Subsequently, the elution was performed by a linear gradient to 59% ethanol in 0.069% w/w sodium di-hydrogen phosphate monohydrate, 2.06% w/w potassium acetate, pH 3.5 during 71 mL (9 CV). The experiment was performed at room temperature.

Separation between L-His¹ Exendin-4(1-39) and D-His¹ Exendin-4(1-39) was obtained and confirmed by retention time analysis, D-His¹ Exendin-4(1-39) eluting before L-His¹ Exendin-4(1-39).

### Example 21

L-His¹ Exendin-4(1-39) and D-His¹ Exendin-4(1-39) were synthesized by standard solid phase synthesis methods using Fmoc chemistry.

A solution of L-His¹ Exendin-4(1-39) containing the related impurity D-His¹ Exendin-4(1-39) were dissolved in water to a total concentration of 1 mg peptide/mL. 8 mL of the solution was loaded to a 7.85 mL column containing 120 Å C18 substituted (octadecyl-dimethyl silyl) silica gel (particle size 15 µm) equilibrated with 15.7 mL of a solvent containing 25% w/w ethanol, 0.13% w/w MES, 2.06% w/w potassium acetate pH 6.7. The column was washed with 3.9 mL equilibration solution. The elution was performed with a isocratic gradient of 34% ethanol during 157 mL (20 CV) followed by a 24 mL (3 CV) linear gradient from 34% to 39% ethanol in 0.13% w/w MES, 2.06% w/w potassium acetate, pH 6.7. Subsequently, the elution was performed by a step gradient to 59% ethanol in 0.13% w/w MES, 2.06% w/w potassium acetate, pH 6.7 maintained for 7.85 mL (1 CV). The experiment was performed at room temperature.

Separation between L-His¹ Exendin-4(1-39) and D-His¹ Exendin-4(1-39) was obtained and confirmed by retention time analysis, D-His¹ Exendin-4(1-39) eluting before L-His¹ Exendin-4(1-39).

## Claims

1. A method for purifying a glucagon-like peptide from a composition comprising said glucagon-like peptide and at least one related impurity, which method is a reversed phase high performance liquid chromatographic process wherein the solvent used for elution is pH-buffered in the range from pH 4 to pH 10, said solvent is pH-buffered so as to prevent pH excursions of more than +/-1.0 pH units from the setpoint during the elution step, and said solvent comprises an alcohol in a concentration from 20% w/w to 60% w/w,
wherein a related impurity is an impurity which has structural resemblance to the target glucagon-like peptide and is selected from the group consisting of a truncated form, an extended form, a deamidated form, an incorrectly folded form, a form with undesired glycosylation, an oxidated form, a form resulting from racemization, a form lacking amino acids in the intra-peptide chain, a form having extra amino acids in the intra-peptide chain and a form wherein an acylation has taken place on another residue than desired.

2. A method according to claim 1, wherein said solvent is pH-buffered in the range from pH 5 to pH 9.

3. A method according to claim 1, wherein said solvent is pH-buffered at a pH which is higher than the isoelectric point of said glucagon-like peptide.

4. A method according to any of the previous claims, wherein said solvent is pH-buffered so as to prevent pH excursions of more than +/-0.5 pH units from the setpoint during the elution step.

5. A method according to any of the previous claims, wherein said alcohol is ethanol.

6. A method according to any of the previous claims, wherein said alcohol is 2-propanol.

7. A method according to any of the previous claims, wherein said alcohol is selected from the group consisting of methanol, 1-propanol and hexylen glycol.

8. A method according to any of the previous claims, wherein the solvent further comprises one or more salts selected from the group consisting of: NaCl, KCl, NH₄Cl, CaCl₂, sodium acetate, potassium acetate and ammonium acetate.

9. A method according to any of the previous claims, wherein the buffer is selected from the group consisting of: Citrate buffer, phosphate buffer, TRIS buffer, borate buffer, carbonate buffer, acetate buffer, ammonium buffer and glycine buffer.

10. A method according to any of the previous claims, wherein said reversed phase high performance liquid chromatographic process is performed using a silica based chromatographic resin.

11. A method according to claim 10, wherein said resin is a substituted silica gel, such as C₄-, C₆-, C₈-, C₁₂-, C₁₆-, C₁₈-, C₂₀-, phenyl- or benzene-substituted silica gel.

12. A method according to any one of claims 1-9, wherein said reversed phase high performance liquid chromatographic process is performed using a chromatographic resin which is a polymeric base material.

13. A method according to any of the previous claims, wherein said related impurity is a truncated form of said glucagon-like peptide.

14. A method according to any of the previous claims, wherein said related impurity is a glycosylated form of said glucagon-like peptide.

15. The method according to any one of the previous claims, wherein said solvent comprises an alcohol in a concentration from 20% w/w to 40% w/w.

16. The method according to any of the previous claims, wherein said glucagon-like peptide is GLP-1, a GLP-1 analogue, a derivative of GLP-1 or a derivative of a GLP-1 analogue.

17. The method according to claim 16, wherein said GLP-1 analogue is selected from the-group. consisting of Arg³⁴-GLP-1(7-37), Gly⁸-GLP-1(7-36)-amide, Gly⁸-GLP-1(7-37), Val⁸-GLP-1 (7-36)-amide, Val⁸-GLP-1 (7-37), Val⁸Asp²²-GLP-1(7-36)-amide, Val⁸Asp²²-GLP-1 (7-37), Val⁸Glu²²-GLP-1(7-36)-amide, Val⁸Glu²²-GLP-1(7-37), Val⁸Lys²²-GLP-1(7-36)-amide, Val⁸Lys²²-GLP-1(7-37), Val⁸Arg²²-GLP-1(7-36)-amide, Val⁸Arg²²-GLP-1(7-37), Val⁸His²²-GLP-1 (7-36)-amide, Val⁸His²²-GLP-1(7-37), Val⁸Trp¹⁹Glu²²-GLP-1(7-37), Val⁸Glu²² Val²⁵-GLP-1 (7-37), Val⁸Tyr¹⁶Glu²²-GLP-1(7-37), Val⁸Trp¹⁶Glu²²-GLP-1(7-37), Val⁸Leu¹⁶Glu²²-GLP-1(7-37), Val⁸Tyr¹⁸Glu²²-GLP-1 (7-37), Val⁸Glu²²His³⁷-GLP-1(7-37), Val⁸Glu²²Ile³³-GLP-1(7-37), Val⁸Trp¹⁶Glu²²Val²⁵Ile³³-GLP-1(7-37), Val⁸Trp¹⁶Glu²²Ile³³-GLP-1(7-37), Val⁸Glu²²Val²⁵Ile³³-GLP-1(7-37), Val⁸Trp¹⁶Glu²²Val²⁵-GLP-1(7-37), analogues thereof and derivatives of any of these.

18. The method according to claim 16, wherein said derivative of GLP-1 or a derivative of a GLP-1 analogue has a lysine residue, such as one lysine, wherein a lipophilic substituent optionally via a spacer is attached to the epsilon amino group of said lysine.

19. The method according to claim 18, wherein said lipophilic substituent has from 8 to 40 carbon atoms, preferably from 8 to 24 carbon atoms, e. g. 12 to 18 carbon atoms.

20. The method according to any one of claims 18-19, wherein said spacer is present and is selected from an amino acid, e. g. beta-Ala, L-Glu, or aminobutyroyl.

21. The method according to any one of the previous claims, wherein said glucagon-like peptide is a DPPIV-protected glucagon-like peptide.

22. The method according to any one of the preceding claims, wherein said glucagon-like peptide is a plasma stable glucagon-like peptide.

23. The method according to claim 16, wherein said derivative of a GLP-1 analogue is Arg³⁴, Lys²⁶(N^{ε}-(γ-Glu(N^{α}-hexadecanoyl)))-GLP-1(7-37).

24. The method according to any one of claims 16-23, wherein said glucagon-like peptide has from 25 to 37 amino acid residues, preferably from 27 to 35 amino acid residues, even more preferable from 29 to 33 amino acid residues.

25. The method according to any one of claims 1-15, wherein said glucagon-like peptide is GLP-2, a GLP-2 analogue, a derivative of GLP-2 or a derivative of a GLP-2 analogue.

26. The method according to claim 25, wherein said derivative of GLP-2 or a derivative of a GLP-2 analogue has a lysine residue, such as one lysine, wherein a lipophilic substituent optionally via a spacer is attached to the epsilon amino group of said lysine.

27. The method according to claim 26, wherein said lipophilic substituent has from 8 to 40 carbon atoms, preferably from 8 to 24 carbon atoms, e.g. 12 to 18 carbon atoms.

28. The method according to any one of claims 26-27, wherein said spacer is present and is selected from an amino acid, e.g. beta-Ala, L-Glu, aminobutyroyl.

29. The method according to any one of claims 25-28, wherein said glucagon-like peptide has from 27 to 39 amino acid residues, preferable from 29 to 37 amino acid residues, even more preferable from 31 to 35 amino acid residues.

30. The method according to claim 25, wherein said glucagon-like peptide is Gly²-GLP-2(1-33).

31. The method according to any one of claims 1-15, wherein said glucagon-like peptide is exendin-4, an exendin-4 analogue, a derivative of exendin-4, or a derivative of an exendin-4 analogue.

32. The method according to claim 31, wherein said glucagon-like peptide is exendin-4.

33. The method according to claim 31, wherein said glucagon-like peptide is ZP-10, i.e.
HGEGTFTSDLSKQMEEEAVRLFIEWLKNGGPSSGAPPSKKKKKK-NH2.

34. The method according to claim 31, wherein said derivative of exendin-4 or derivative of an exendin-4 analogue is acylated or pegylated.

35. The method according to claim 31, wherein said derivative of exendin-4 or derivative of an exendin-4 analogue has a lysine residue, such as one lysine, wherein a lipophilic substituent optionally via a spacer is attached to the epsilon amino group of said lysine.

36. The method according to claim 35, wherein said lipophilic substituent has from 8 to 40 carbon atoms, preferably from 8 to 24 carbon atoms, e.g. 12 to 18 carbon atoms.

37. The method according to any one of claims 35-36, wherein said spacer is present and is selected from an amino acid, e.g. beta-Ala, L-Glu, or aminobutyroyl.

## Patentansprüche

1. Verfahren zum Reinigen eines glucagonartigen Peptids aus einer Zusammensetzung, umfassend das glucagonartige Peptid und mindestens eine damit verbundene Verunreinigung, wobei das Verfahren ein Umkehrphasenhochleistungsflüssigchromatographieverfahren ist, wobei das zur Elution verwendete Lösungsmittel im Bereich von pH 4 bis pH 10 pH-gepuffert ist, wobei das Lösungsmittel pH-gepuffert ist, um pH-Abweichungen von mehr als +/- 1,0 pH-Einheiten vom Einstellpunkt während des Elutionsschritts zu verhindern, und wobei das Lösungsmittel einen Alkohol in einer Konzentration von 20 Gew.-% bis 60 Gew.-% umfasst,
wobei eine damit verbundene Verunreinigung eine Verunreinigung ist, die eine strukturelle Ähnlichkeit mit dem glucagonartigen Zielpeptid aufweist und ausgewählt ist aus der Gruppe, bestehend aus einer verkürzten Form, einer verlängerten Form, einer deamidierten Form, einer nicht korrekt gefalteten Form, einer Form mit unerwünschter Glycosylierung, einer oxidierten Form, einer aus Racemisierung erhaltenen Form, einer Form, der Aminosäuren in der Intrapeptidkette fehlen, einer Form, die zusätzliche Aminosäuren in der Intrapeptidkette aufweist und einer Form, wobei eine Acylierung an einem anderem Rest als gewünscht stattfand.

2. Verfahren nach Anspruch 1, wobei das Lösungsmittel im Bereich von pH 5 bis pH 9 pH-gepuffert ist.

3. Verfahren nach Anspruch 1, wobei das Lösungsmitte bei einem pH-Wert pH-gepuffert ist, der höher als der isoelektrische Punkt des glucagonartigen Peptids ist.

4. Verfahren nach einem der vorangehenden Ansprüche, wobei das Lösungsmittel pH-gepuffert ist, um pH-Abweichungen von mehr als +/-0,5 pH-Einheiten vom Einstellpunkt während des Elutionsschritts zu verhindern.

5. Verfahren nach einem der vorangehenden Ansprüche, wobei der Alkohol Ethanol ist.

6. Verfahren nach einem der vorangehenden Ansprüche, wobei der Alkohol 2-Propanol ist.

7. Verfahren nach einem der vorangehenden Ansprüche, wobei der Alkohol ausgewählt ist aus der Gruppe, bestehend aus Methanol, 1-Propanol und Hexylenglycol.

8. Verfahren nach einem der vorangehenden Ansprüche, wobei das Lösungsmittel des Weiteren ein oder mehrere Salze umfasst, ausgewählt aus der Gruppe, bestehend aus: NaCl, KCl, NH₄Cl, CaCl₂, Natriumacetat, Kaliumacetat und Ammoniumacetat.

9. Verfahren nach einem der vorangehenden Ansprüche, wobei der Puffer ausgewählt ist aus der Gruppe, bestehend aus: Zitratpuffer, Phoshphatpuffer, TRIS-Puffer, Boratpuffer, Carbonatpuffer, Acetatpuffer, Ammoniumpuffer und Glycinpuffer.

10. Verfahren nach einem der vorangehenden Ansprüche, wobei das Umkehrphasenhochleistungsflüssigchromatographieverfahren unter Verwendung eines Chromatographieharzes auf Silicabasis durchgeführt wird.

11. Verfahren nach Anspruch 10, wobei das Harz ein substituiertes Silicagel, wie C₄-, C₆-, C₈-, C₁₂-, C₁₆-, C₁₈-, C₂₀-, phenyl- oder benzolsubstituiertes Silicagel ist.

12. Verfahren nach einem der Ansprüche 1-9, wobei das Umkehrphasenhochleistungsflüssigchromatographieverfahren unter Verwendung eines Chromatographieharzes, bei dem es sich um ein Material auf Polymerbasis handelt, durchgeführt wird.

13. Verfahren nach einem der vorangehenden Ansprüche, wobei die damit verbundene Verunreinigung eine verkürzte Form des glucagonartigen Peptids ist.

14. Verfahren nach einem der vorangehenden Ansprüche, wobei die damit verbundene Verunreinigung eine glycosylierte Form des glucagonartigen Peptids ist.

15. Verfahren nach einem der vorangehenden Ansprüche, wobei das Lösungsmittel einen Alkohol in einer Konzentration von 20 Gew.-% bis 40 Gew.-% umfasst.

16. Verfahren nach einem der vorangehenden Ansprüche, wobei das glucagonartige Peptid ein GLP-1, ein GLP-1-Analogon, ein Derivat von GLP-1 oder ein Derivat eines GLP-1-Analogons ist.

17. Verfahren nach Anspruch 16, wobei das GLP-1-Analogon ausgewählt ist aus der Gruppe bestehend aus Arg³⁴-GLP-1(7-37), Gly⁸-GLP-1(7-36)-amid, Gly⁸-GLP-1(7-37), Val⁸- GLP-1(7-36)-amid, Val⁸-GLP-1(7-37), Val⁸Asp²²-GLP-1(7-36)-amid, Val⁸Asp²²-GLP-1(7-37), Val⁸Glu²²-GLP-1(7-36)-amid, Val⁸Glu²²-GLP-1(7-37), Val⁸Lys²²-GLP-1(7-36)-amid, Val⁸Lys²²-GLP-1(7-37), Val⁸Arg²²-GLP-1(7-36)-amid, Val⁸Arg²²-GLP-1(7-37), Val⁸HiS²² -GLP-1(7-36)-amid, Val⁸His²²-GLP-1(7-37), Val⁸Trp¹⁹Glu²²-GLP-1(7-37), Val⁸Glu²²Val²⁵-GLP-1(7-37), Val⁸Tyr¹⁶Glu²²-GLP-1(7-37), Val⁸Trp¹⁶Glu²²-GLP-1(7-37), Val⁸Leu¹⁶Glu²²-GLP-1(7-37), Val⁸Tyr¹⁸Glu²²-GLP-1(7-37), Val⁸Glu²²His³⁷-GLP-1(7-37), Val⁸Glu²²Ile³³-GLP-1(7-37), Val⁸Trp¹⁶Glu²²Val²¹Ile³³-GLP-1(7-37), Val⁸Trp¹⁶Glu²²Ile³³-GLP-1(7-37), Val⁸Glu²² Val²¹Ile³³-GLP-1(7-37), Val⁸Trp¹⁸Glu²²Val²⁵-GLP-1(7-37), Analoge davon und Derivate von einer von diesen.

18. Verfahren nach Anspruch 16, wobei das Derivat von GLP-1 oder ein Derivat von einem GLP-1-Analogon einen Lysinrest, wie ein Lysin, aufweist, wobei ein lipophiler Substituent wahlweise über einen Abstandhalter an die Epsilonaminogruppe des Lysins angelagert ist.

19. Verfahren nach Anspruch 18, wobei der lipophile Substituent 8 bis 40 Kohlenstoffatome, vorzugsweise 8 bis 24 Kohlenstoffatome, z.B. 12 bis 18 Kohlenstoffatome aufweist.

20. Verfahren nach einem der Ansprüche 18-19, wobei der Abstandhalter vorliegt und aus einer Aminosäure, z.B. beta-Ala, L-Glu oder Aminobutyroyl ausgewählt ist.

21. Verfahren nach einem der vorangehenden Ansprüche, wobei das glucagonartige Peptid ein DPPIV-geschütztes glucagonartiges Peptid ist.

22. Verfahren nach einem der vorangehenden Ansprüche, wobei das glucagonartige Peptid ein plasmastabiles glucagonartiges Peptid ist.

23. Verfahren nach Anspruch 16, wobei das Derivat eines GLP-1-Analogons Arg³⁴,Lys²⁶(N^{ε}-(γ-GluN^{α}-hexadecanoyl)))-GLP-1(7-37) ist.

24. Verfahren nach einem der Ansprüche 16-23, wobei das glucagonartige Peptid 25 bis 37 Aminosäurereste, vorzugsweise 27 bis 35 Aminosäurereste, noch stärker bevorzugt 29 bis 33 Aminosäurereste aufweist.

25. Verfahren nach einem der Ansprüche 1-15, wobei das glucagonartige Peptid GLP-2, ein GPL-2-Analogon, ein Derivat von GLP-2 oder ein Derivat eines GLP-2-Analogons ist.

26. Verfahren nach Anspruch 25, wobei das Derivat von GLP-2 oder ein Derivat von einem GLP-2-Analogon einen Lysinrest, wie ein Lysin, aufweist, wobei ein lipophiler Substituent wahlweise über einen Abstandhalter an die Epsilonaminogruppe des Lysins angelagert ist.

27. Verfahren nach Anspruch 26, wobei der lipophile Substituent 8 bis 40 Kohlenstoffatome, vorzugsweise 8 bis 24 Kohlenstoffatome, z.B. 12 bis 18 Kohlenstoffatome aufweist.

28. Verfahren nach einem der Ansprüche 26-27, wobei der Abstandhalter vorliegt und aus einer Aminosäure, z.B. beta-Ala, L-Glu oder Aminobutyroyl ausgewählt ist.

29. Verfahren nach einem der Ansprüche 25-28, wobei das glucagonartige Peptid 27 bis 39 Aminosäurereste, vorzugsweise 29 bis 37 Aminosäurereste, noch stärker bevorzugt 31 bis 35 Aminosäurereste aufweist.

30. Verfahren nach Anspruch 25, wobei das glucagonartige Peptid Gly²-GLP-2(1-33) ist.

31. Verfahren nach einem der Ansprüche 1-15, wobei das glucagonartige Peptid Exendin-4, ein Exendin-4-Analogon, ein Derivat von Exendin-4 oder ein Derivat eines Exendin-4-Analogons ist.

32. Verfahren nach Anspruch 31, wobei das glucagonartige Peptid Exendin-4 ist.

33. Verfahren nach Anspruch 31, wobei das glucagonartige Peptid ZP-10, d.h. HGEGTFTSDLSKQMEEEAVRLFIEWLKNGGPSSGAPPSKKKKKK-NH2 ist.

34. Verfahren nach Anspruch 31, wobei das Derivat von Exendin-4 oder Derivat eines Exendin-4-Analogons acyliert oder pegyliert ist.

35. Verfahren nach Anspruch 31, wobei das Derivat von Exendin-4 oder Derivat eines Exendin-4-Analogons einen Lysinrest, wie ein Lysin, aufweist, wobei ein lipophiler Substituent wahlweise über einen Abstandhalter an die Epsilonaminogruppe des Lysins angelagert ist.

36. Verfahren nach Anspruch 35, wobei der lipophile Substituent 8 bis 40 Kohlenstoffatome, vorzugsweise 8 bis 24 Kohlenstoffatome, z.B. 12 bis 18 Kohlenstoffatome aufweist.

37. Verfahren nach einem der Ansprüche 35-36, wobei der Abstandhalter vorliegt und ausgewählt ist aus einer Aminosäure, z.B. beta-Ala, L-Glu oder Aminobutyroyl.

## Revendications

1. Procédé pour purifier un glucagon-like peptide, à partir d'une composition comprenant ledit glucagon-like peptide et au moins une impureté apparentée, lequel procédé est un procédé de chromatographie en phase liquide hautes performances en phase inversée, dans lequel le solvant utilisé pour l'élution a son pH tamponné dans la plage de pH 4 à pH 10, ledit solvant a son pH tamponné de façon à empêcher que le pH ne varie de plus de +/- 1,0 unité de pH à partir du point de réglage au cours de l'étape d'élution, et ledit solvant comprend un alcool à une concentration de 20 à 60% p/p, l'impureté apparentée étant une impureté ayant une ressemblance structurale avec le glucagon-like peptide cible et est choisie dans le groupe consistant en une forme tronquée, une forme étendue, une forme désamidée, une forme incorrectement repliée, une forme présentant une glycosylation indésirable, une forme oxydée, une forme résultant d'une racémisation, une forme manquant d'acides aminés dans la chaîne intrapeptidique, une forme ayant des acides aminés supplémentaires dans la chaîne intrapeptidique et une forme dans laquelle une acylation a eu lieu sur un résidu différent de celui qui est souhaité.

2. Procédé selon la revendication 1, dans lequel ledit solvant a son pH tamponné dans la plage de pH 5 à 9.

3. Procédé selon la revendication 1, dans lequel ledit solvant a son pH tamponné à une valeur supérieure au point isoélectrique dudit glucagon-like peptide.

4. Procédé selon l'une quelconque des revendications précédentes, dans lequel ledit solvant a son pH tamponné de façon à empêcher une variation du pH supérieure à +/-0,5 unité de pH à partir du point de réglage au cours de l'étape d'élution.

5. Procédé selon l'une quelconque des revendications précédentes, dans lequel ledit alcool est l'éthanol.

6. Procédé selon l'une quelconque des revendications précédentes, dans lequel ledit alcool est le 2-propanol.

7. Procédé selon l'une quelconque des revendications précédentes, dans lequel ledit alcool est choisi dans le groupe consistant en le méthanol, le 1-propanol et l'hexylèneglycol.

8. Procédé selon l'une quelconque des revendications précédentes, dans lequel le solvant comprend en outre un ou plusieurs sels choisis dans le groupe consistant en NaCl, KCl, NH₄Cl, CaCl₂, l'acétate de sodium, l'acétate de potassium et l'acétate d'ammonium.

9. Procédé selon l'une quelconque des revendications précédentes, dans lequel le tampon est choisi dans le groupe consistant en le tampon citrate, le tampon phosphate, le tampon TRIS, le tampon borate, le tampon carbonate, le tampon acétate, le tampon ammonium et le tampon glycine.

10. Procédé selon l'une quelconque des revendications précédentes, dans lequel ledit procédé de chromatographie en phase liquide hautes performances en phase inversée est mis en oeuvre par utilisation d'une résine pour chromatographie à base de silice.

11. Procédé selon la revendication 10, dans lequel ladite résine est un gel de silice substitué, tel qu'un gel de silice à substitution C₄, C₆, C₈, C₁₂, C₁₆, C₁₈, C₂₀, phényle ou benzène.

12. Procédé selon l'une quelconque des revendications 1 à 9, dans lequel ledit procédé de chromatographie en phase liquide hautes performances en phase inversée est mis en oeuvre par utilisation d'une résine pour chromatographie qui est un matériau à base polymère.

13. Procédé selon l'une quelconque des revendications précédentes, dans lequel ladite impureté apparentée est une forme tronquée dudit glucagon-like peptide.

14. Procédé selon l'une quelconque des revendications s précédentes, dans lequel ladite impureté apparentée est une forme glycosylée dudit glucagon-like peptide.

15. Procédé selon l'une quelconque des revendications précédentes, dans lequel ledit solvant comprend un alcool à une concentration de 20 à 40% p/p.

16. Procédé selon l'une quelconque des revendications précédentes, dans lequel ledit glucagon-like peptide est le GLP-1, un analogue du GLP-1, un dérivé du GLP-1 ou un dérivé d'un analogue du GLP-1.

17. Procédé selon la revendication 16, dans lequel ledit analogue du GLP-1 est choisi dans le groupe consistant en Arg³⁴-GLP-1(7-37), Gly⁸-GLP-1(7-36)-amide, Gly⁸-GLP-1(7-37) , Val⁸-GLP-1(7-36)-amide, Val⁸-GLP-1(7-37), Val⁸Asp²²-GLP-1(7-36)-amide, Val⁸Asp²²-GLP-1(7-37), Val⁸Glu²²-GLP-1(7-36) -amide, Val⁸Glu²²-GLP-1(7-37), Val³Lys²²-GLP-1(7-36)-amide, Val⁸Lys²²-GLP-1(7-37), Val⁸Arg²²-GLP-1(7-36) -amide, Val⁸Arg²²-GLP-1(7-37), Val⁸His²²-GLP-1(7-36) -amide, Val⁸His²²-GLP-1(7-37), Val⁸Trp¹⁹Glu²²-GLP-1(7-37), Val⁸Glu²²Val²⁵-GLP-1(7-37), Val⁸Tyr¹⁶Glu²²-GLP-1(7-37), Val⁸Trp¹⁶Glu²²-GLP-1(7-37), Val⁸Leu¹⁶Glu²²-GLP-1(7-37), Val⁸Tyr¹⁸Glu²²-GLP-1(7-37), Val⁸Glu²²His³⁷-GLP-1(7-37), Val⁸Glu²²Ile³³-GLP-1(7-37), Val⁸Trp¹⁶Glu²²Val²⁵Ile³³-GLP-1(7-37), Val⁸Trp¹⁶Glu²²Ile³³-GLP-1(7-37), Val⁸Glu²²Val²⁵Ile³³ -GLP-1(7-37), Val⁸Trp¹⁶Glu²²val²⁵-GLP-1(7-37), leurs analogues, et les dérivés de l'un quelconque d'entre eux.

18. Procédé selon la revendication 16, dans lequel ledit dérivé du GLP-1 ou dérivé d'un analogue du GLP-1 possède un résidu de lysine, tel qu'une lysine, un substituant lipophile étant en option fixé par l'intermédiaire d'un espaceur au groupe epsilon-amino de ladite lysine.

19. Procédé selon la revendication 18, dans lequel ledit substituant lipophile possède de 8 à 40 atomes de carbone, de préférence de 8 à 24 atomes de carbone, par exemple 12 à 18 atomes de carbone.

20. Procédé selon l'une quelconque des revendications 18-19, dans lequel ledit espaceur est présent et est choisi parmi un acide aminé, par exemple bêta-Ala, L-Glu, ou le groupe aminobutyroyle.

21. Procédé selon l'une quelconque des revendications précédentes, dans lequel ledit glucagon-like peptide est un glucagon-like peptide protégé par la DPPIV.

22. Procédé selon l'une quelconque des revendications précédentes, dans lequel ledit glucagon-like peptide est un glucagon-like peptide plasma-stable.

23. Procédé selon la revendication 16, dans lequel ledit dérivé d'un analogue du GLP-1 est Arg³⁴Lys²⁶(N^{ε}-(γ-Glu(N^{α}-hexadécanoyl)))-GLP-1(7-37).

24. Procédé selon l'une quelconque des revendications 16 à 23, dans lequel ledit glucagon-like peptide possède de 25 à 37 résidus d'acides aminés, de préférence de 27 à 35 résidus d'acides aminés et d'une manière encore plus préférée de 29 à 33 résidus d'acides aminés.

25. Procédé selon l'une quelconque des revendications 1 à 15, dans lequel ledit glucagon-like peptide est le GLP-2, un analogue du GLP-2, un dérivé du GLP-2 ou un dérivé d'un analogue du GLP-2.

26. Procédé selon la revendication 25, dans lequel ledit dérivé du GLP-2, ou dérivé d'un analogue du dérivé GLP-2, possède un résidu de lysine tel qu'une lysine, un substituant lipophile étant en option fixé par l'intermédiaire d'un espaceur au groupe epsilon-amino de ladite lysine.

27. Procédé selon la revendication 26, dans lequel ledit substituant lipophile a de 8 à 40 atomes de carbone, de préférence de 8 à 24 atomes de carbone, par exemple 12 à 18 atomes de carbone.

28. Procédé selon l'une quelconque des revendications 26-27, dans lequel ledit espaceur est présent et est choisi parmi un acide aminé, par exemple bêta-Ala, L-Glu, un groupe aminobutyroyle.

29. Procédé selon l'une quelconque des revendications 25-28, dans lequel ledit glucagon-like peptide a de 27 à 39 résidus d'acides aminés, de préférence de 29 à 37 résidus d'acides aminés et d'une manière encore plus préférée de 31 à 35 résidus d'acides aminés.

30. Procédé selon la revendication 25, dans lequel ledit glucagon-like peptide est le Gly²GLP-2(1-33).

31. Procédé selon l'une quelconque des revendications 1 à 15, dans lequel ledit glucagon-like peptide est l'exendine-4, un analogue de l'exendine-4, un dérivé de l'exendine-4 ou un analogue de l'exendine-4.

32. Procédé selon la revendication 31, dans lequel ledit glucagon-like peptide est l'exendine-4.

33. Procédé selon la revendication 31, dans lequel ledit glucagon-like peptide est le ZP-10, à savoir :
**HGEGTFTSDLSKQMEEEAVRLFIEWLKNGGPSSGAPPSKKKKKK-NH2.**

34. Procédé selon la revendication 31, dans lequel ledit dérivé de l'exendine-4 ou dérivé d'un analogue de l'exendine-4 est acylé ou pégylé.

35. Procédé selon la revendication 31, dans lequel ledit dérivé de l'exendine-4 ou dérivé d'un analogue de l'exendine-4 possède un résidu de lysine, tel qu'une lysine, un substituant lipophile étant en option fixé par l'intermédiaire d'un espaceur ou groupe epsilon-amino de ladite lysine.

36. Procédé selon la revendication 35, dans lequel ledit substituant lipophile a de 8 à 40 atomes de carbone, de préférence de 8 à 24 atomes de carbone, par exemple 12 à 18 atomes de carbone.

37. Procédé selon l'une quelconque des revendications 35-36, dans lequel ledit espaceur est présent et est choisi parmi un acide aminé, par exemple bêta-Ala, L-Glu, ou le groupe aminobutyroyle.
